(19) Europäisches Patentamt
European Patent Office
Office européen des brevets

(11) Veröffentlichungsnummer: **0 359 979 B1**

# (12) EUROPÄISCHE PATENTSCHRIFT

(45) Veröffentlichungstag der Patentschrift: **30.12.92**

(51) Int. Cl.[5]: **C07D 317/72**, C07D 327/04, C07D 405/06, C07D 413/06, C07D 411/06, A01N 43/28, A01N 43/30

(21) Anmeldenummer: **89114788.6**

(22) Anmeldetag: **10.08.89**

Die Akte enthält technische Angaben, die nach dem Eingang der Anmeldung eingereicht wurden und die nicht in dieser Patentschrift enthalten sind.

## (54) Aminomethylheterocyclen, Verfahren zu ihrer Herstellung, ihre Verwendung in Schädlingsbekämpfungsmitteln und neue Zwischenprodukte.

(30) Priorität: **23.08.88 DE 3828545**

(43) Veröffentlichungstag der Anmeldung: **28.03.90 Patentblatt 90/13**

(45) Bekanntmachung des Hinweises auf die Patenterteilung: **30.12.92 Patentblatt 92/53**

(84) Benannte Vertragsstaaten: **BE CH DE FR GB IT LI NL**

(56) Entgegenhaltungen:
**EP-A- 0 097 822**
**EP-A- 0 131 793**
**EP-A- 0 281 842**
**DE-A- 1 965 321**

**CHEMICAL AND PHARAMCEUTICAL BULLETIN, Band 32, Nr. 3, 1984, Seiten 967-976; S. SUGAI et al.: "Studies on Spasmolytics. I. Synthesis and Spasmolytic Activities of 4-Acyloxy-1-(1,3-dioxolan-4-ylmethyl)piperidines"**

(73) Patentinhaber: **BAYER AG**
**W-5090 Leverkusen 1 Bayerwerk(DE)**

(72) Erfinder: **Krämer, Wolfgang, Dr.**
**Rosenkranz 25**
**W-5093 Burscheid 2(DE)**
Erfinder: **Weissmüller, Joachim, Dr.**
**Carl-Langhans-Strasse 53**
**W-4019 Monheim(DE)**
Erfinder: **Berg, Dieter, Dr.**
**Gellertweg 27**
**W-5600 Wuppertal 1(DE)**
Erfinder: **Brandes, Wilhelm, Dr.**
**Eichendorffstrasse 3**
**W-5653 Leichlingen(DE)**
Erfinder: **Dutzmann, Stefan, Dr.**
**Leinenweberweg 33**
**W-4000 Düsseldorf 13(DE)**

EP 0 359 979 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue Aminomethylheterocyclen, mehrere Verfahren zu ihrer Herstellung, ihre Verwendung in Schädlingsbekämpfungsmitteln und neue Zwischenprodukte.

Es ist bekannt, daß bestimmte Aminomethyldioxolane, wie beispielsweise die Verbindung 2-Isobutyl-2-methyl-4-(1-piperidinylmethyl)-1,3-dioxolan oder die Verbindung 2-Methyl-2-nonyl-4-di-n-butylaminomethyl-1,3-dioxolan oder die Verbindung 2-(2-Cyclohexylmethyl-2-propyl)-2-methyl-4-(1-piperidinylmethyl)-1,3-dioxolan oder die Verbindung 2-(2-Cyclohexylmethyl-2-propyl)-2-methyl-4-(1-perhydroazepinylmethyl)-1,3-dioxolan fungizide Eigenschaften besitzen (vergl. z.B. EP 97 822).

Weiterhin sind 5-Aminomethyl-1,3-oxathiolane bekannt, die für den Gebrauch als Schädlingsbekämpfungsmittel geeignet sind (vgl. z.B. EP-A 131 793).

Weiterhin sind sehr naheliegende 1,3-Dioxolane und 1,3-Oxathiolane beschrieben; dieser Stand der Technik ist durch einen Disclaimer in der vorliegenden Anmeldung ausgeschlossen worden. Es sei noch vermerkt, daß dieses Dokument , die EP-A 281 842, unter Artikel 54(3) EPÜ fällt.

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue Aminomethylheterocyclen der allgemeinen Formel (I),

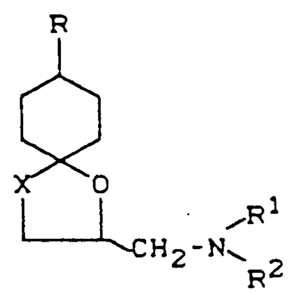

(I)

in welcher

X für Sauerstoff oder Schwefel steht,

R für Cycloalkyl mit 3 bis 7 Kohlenstoffatomen, für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht oder für einen Rest

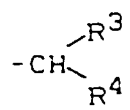

steht,

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder

verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen,

$R^3$ für Wasserstoff, für geradkettiges oder verzweigtes Alkyl mit 1 bis 12 Kohlenstoffatomen oder für gegebenenfalls einfach bis mehrfach, gleich oder verschieden durch geradkettiges oder verzweigtes Alkyl mit 1 bis 12 Kohlenstoffatomen Substituiertes Aryl mit 6 bis 10 Kohlenstoffatomen steht und

$R^4$ für geradkettiges oder verzweigtes Alkyl mit 1 bis 12 Kohlenstoffatomen, für Cycloalkyl mit 3 bis 7 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht,

wobei jedoch $R^3$ und $R^4$ nicht gleichzeitig für Methyl stehen dürfen, und deren Säureadditionssalze gefunden.

Die Verbindungen der Formel (I) können als geometrische und/oder optische Isomere oder Isomerengemische unterschiedlicher Zusammensetzung vorliegen, Sowohl die reinen Isomeren als auch die Isomerengemische werden erfindungsgemäß beansprucht, wenn von Verbindungen der Formel (I) die Rede ist.

Weiterhin wurde gefunden, daß man die neuen Aminomethylheterocyclen der allgemeinen Formel (I),

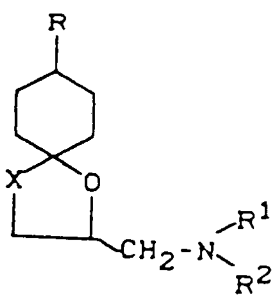

(I)

in welcher

X für Sauerstoff oder Schwefel steht,

R für Cycloalkyl mit 3 bis 7 Kohlenstoffatomen, für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht oder für einen Rest

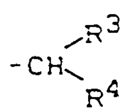

steht,

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen in Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen,

$R^3$ für Wasserstoff, für geradkettiges oder verzweigtes Alkyl mit 1 bis 12 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht und

$R^4$ für geradkettiges oder verzweigtes Alkyl mit 1 bis 12 Kohlenstoffatomen, für Cycloalkyl mit 3 bis 7 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht,
wobei jedoch $R^3$ und $R^4$ nicht gleichzeitig für Methyl stehen dürfen, und deren Säureadditionssalze erhält, wenn man

(a) substituierte Heterocyclen der Formel (II),

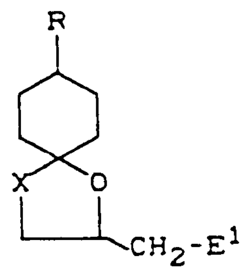

(II)

in welcher
R und X die oben angegebene Bedeutung haben und
$E^1$ für eine elektronenanziehende Abgangsgruppe steht,
mit einen der Formel (III),

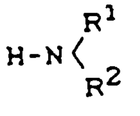

(III)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt, oder wenn man

(b) die nach Verfahren (a) erhältlichen Aminomethylheterocyclen der Formel (Ia),

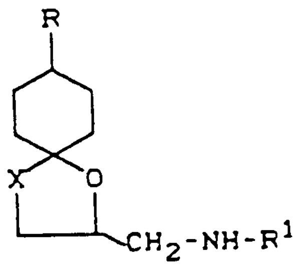

(Ia)

in welcher

R, $R^1$ und X die oben angegebene Bedeutung haben,

mit Alkylierungsmitteln der Formel (IV),

$R^{2-1}$-$E^2$ (IV)

in welcher

$R^{2-1}$ die oben für $R^2$ angegebene Bedeutung, ausgenommen Wasserstoff und Aryl hat und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

Schließlich wurde gefunden, daß die neuen Aminomethylheterocyclen der allgemeinen Formel (I) eine Wirkung gegen Schädlinge, insbesondere gegen pilzliche Schädlinge besitzen.

Überraschenderweise zeigen die erfindungsgemäßen Aminomethylheterocyclen der allgemeinen Formel (I) u.a. eine bessere fungizide Wirksamkeit als die aus dem Stand der Technik bekannten Aminomethyldioxolane, wie beispielsweise die Verbindung 2-Isobutyl-2-methyl-4-(1-piperidinylmethyl)-1,3-dioxolan oder die Verbindung 2-Methyl-2-nonyl-4-di-n-butylaminomethyl-1,3-dioxolan oder die Verbindung 2-(2-Cyclohexylmethyl-2-propyl)-2-methyl-4-(1-piperidinylmethyl)-1,3-dioxolan oder die Verbindung 2-(2-Cyclohexylmethyl-2-propyl)-2-methyl-4-(1-perhydroazepinylmethyl)-1,3-dioxolan, welche chemisch und wirkungsmäßig naheliegende Verbindungen sind.

Die erfindungsgemäßen Aminomethylheterocyclen sind durch die Formel (I) allgemein definiert. Bevorzugt sind Verbindungen der Formel (I), in welcher

X für Sauerstoff oder Schwefel steht,

R für Cyclopentyl, Cyclohexyl oder für gegebenenfalls ein- bis dreifach, gleich oder verschieden durch Methyl, Ethyl, n- oder i-Propyl, n-, i-, s- oder t-Butyl substituiertes Phenyl steht oder für einen Rest

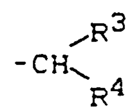

steht,

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls einbis fünffach, gleich oder verschieden durch Fluor, Chlor, Brom, Methyl, Ethyl, n- oder i-Propyl, n-, i-, s- oder t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

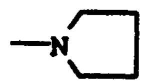 ; 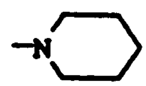 ; 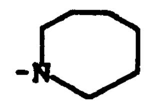 ; 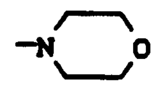

oder 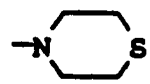

stehen, wobei als Substituenten jeweils infrage kommen: Methyl, Ethyl oder Hydroxymethyl,

und

$R^3$ für Wasserstoff, Methyl, Ethyl, n- oder i-Propyl, für jeweils geradkettiges oder verzweigtes Butyl, Pentyl oder Hexyl steht oder für gegebenenfalls ein- bis dreifach, gleich oder verschieden durch Methyl, Ethyl, n- oder i-Propyl, n-, i-, s- oder t-Butyl substituiertes Phenyl steht und

$R^4$ für Methyl, Ethyl, n- oder i-Propyl, für jeweils geradkettiges oder verzweigtes Butyl, Pentyl oder Hexyl steht, für Cyclopentyl, Cyclohexyl oder für gegebenenfalls ein- bis dreifach, gleich oder verschieden durch Methyl, Ethyl, n- oder i-Propyl, n-, i-, s- oder t-Butyl substituiertes Phenyl steht,

wobei jedoch $R^3$ und $R^4$ nicht gleichzeitig für Methyl stehen dürfen.

Ganz besonders bevorzugt sind Verbindungen der Formel (I), bei welchen

X für Sauerstoff oder Schwefel steht,

R für Cyclohexyl, für Phenyl oder für einen Rest

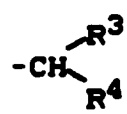

steht,

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

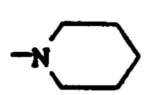 ; 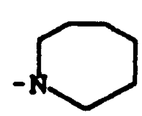 ; 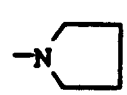 oder 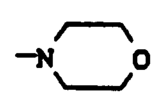

stehen,

wobei als Substituenten jeweils infrage kommen: Methyl, Ethyl oder Hydroxymethyl und

$R^3$ für Wasserstoff, Methyl, Ethyl, n- oder i-Propyl, n-, i-, s- oder t-Butyl oder Phenyl steht und

$R^4$ für Methyl, Ethyl, n- oder i-Propyl, n-, i-, s-oder t-Butyl, für Cyclohexyl oder für Phenyl steht,

wobei jedoch $R^3$ und $R^4$ nicht gleichzeitig für Methyl stehen dürfen.

Bevorzugte erfindungsgemäße Verbindungen sind auch Additionsprodukte aus Säuren und denjenigen Aminomethylheterocyclen der Formel (I), in denen die Substituenten X, R, $R^1$ und $R^2$ die Bedeutungen haben, die bereits für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, und Sulfonsäuren, wie p-Toluolsulfonsäure und 1,5-Naphthalindisulfonsäure und ferner Saccharin.

Im einzelnen seien außer den bei den Herstellungsbeispielen genannten Verbindungen die folgenden Aminomethylheterocyclen der allgemeinen Formel (I) genannt:

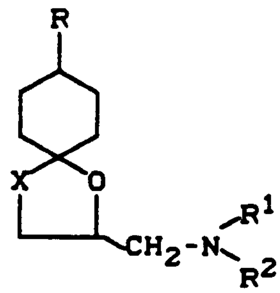

(I)

| R | X | 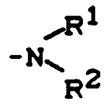 |
|---|---|---|
| 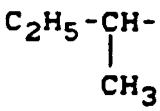 | O | 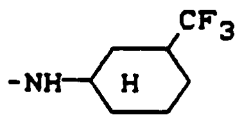 |
| 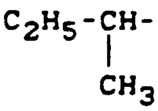 | O | 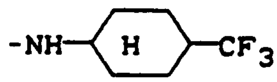 |
| 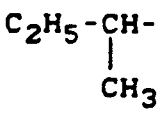 | O | 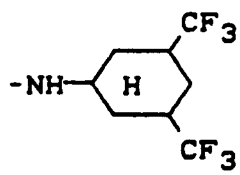 |
| 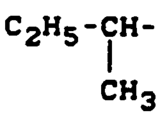 | O | 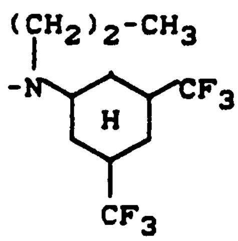 |

| R | X | 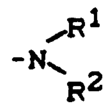 |
|---|---|---|
| 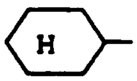 | O | 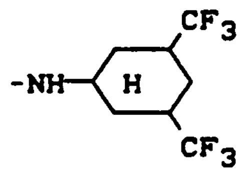 |
| 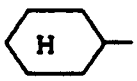 | O | 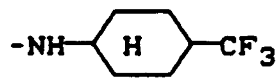 |
| 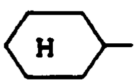 | O | 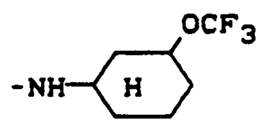 |
| 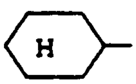 | O | 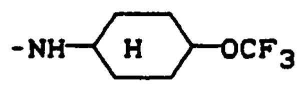 |
| 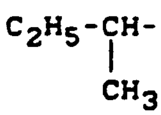 | S | 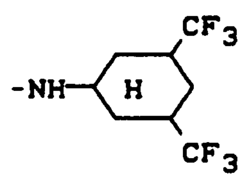 |
| 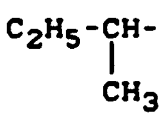 | S | 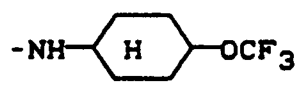 |
| 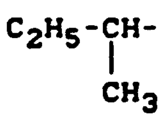 | O | 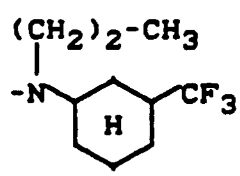 |

Verwendet man beispielsweise 8-s-Butyl-2-chlormethyl-1,4-dioxaspiro[4,5]decan und Piperidin als Ausgangsstoffe, so läßt sich der Reaktionsablauf des erfindungsgemäßen Verfahrens (a) durch das folgende Formelschema darstellen:

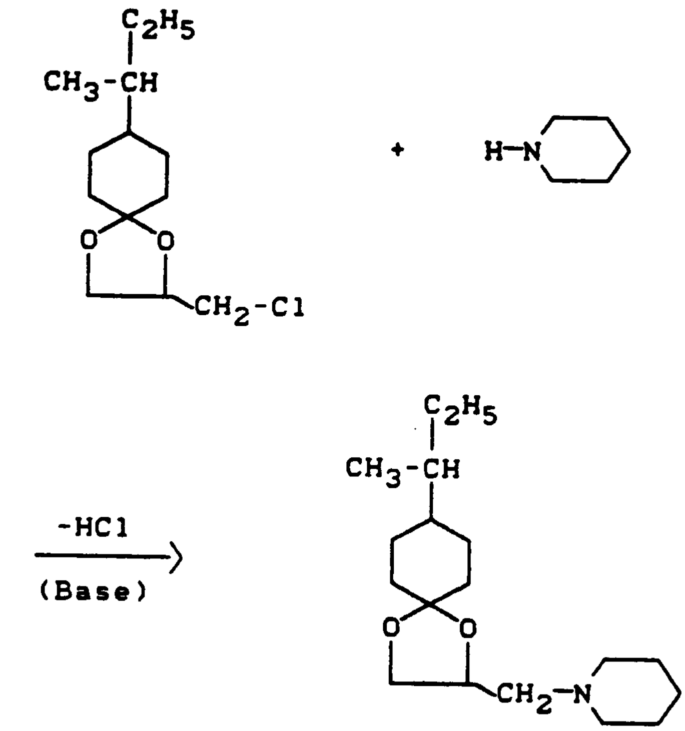


Verwendet man beispielsweise 8-Cyclohexyl-2-methylaminomethyl-1,4-dioxaspiro[4,5]decan und Allylbromid als Ausgangsstoffe, so läßt sich der Reaktionsablauf des erfindungsgemäßen Verfahrens (b) durch das folgende Formelschema darstellen:

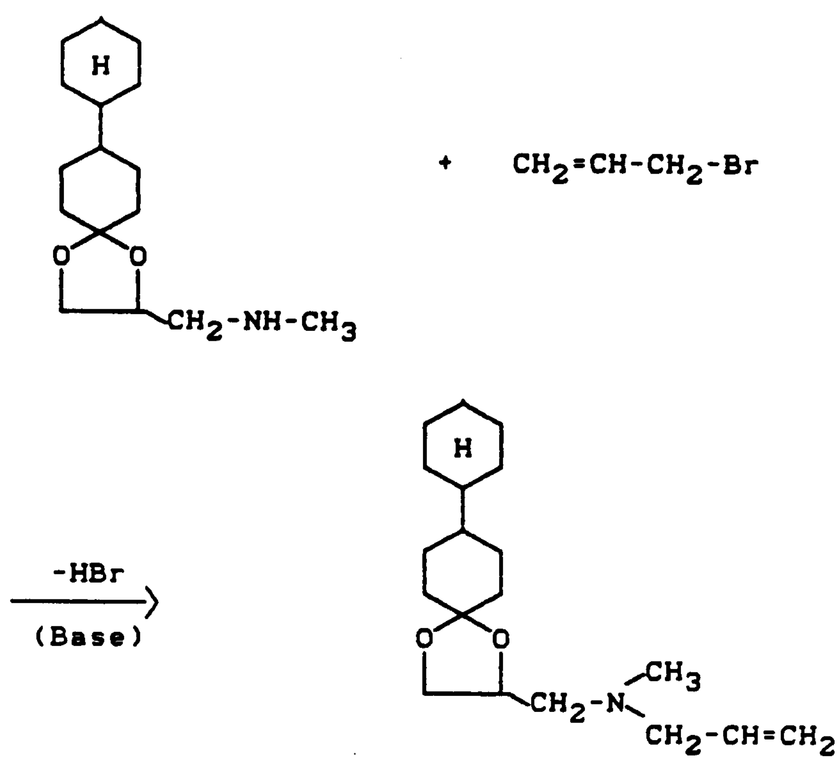


Die zur Durchführung des erfindungsgemäßen Verfahrens (a) als Ausgangsstoffe benötigten substituierten Heterocyclen sind durch die Formel (II) allgemein definiert. In dieser Formel (II) stehen R und X vorzugsweise für diejenigen Reste, die bereits im Zusammenhang mit der Beschreibung der erfindungsgemäßen Stoffe der Formel (I) für diese Substituenten genannt wurden.

$E^1$ steht vorzugsweise für Halogen, insbesondere für Iod, Chlor oder Brom, oder für gegebenenfalls durch Halogen, wie Fluor, Chlor, Brom oder Iod substituiertes Alkylsulfonyloxy oder für gegebenenfalls u.a. durch Alkyl mit 1 bis 4 Kohlenstoffatomen substituiertes Arylsulfonyloxy, wie beispielsweise Methansulfonyloxy, Trifluormethansulfonyloxy oder p-Toluolsulfonyloxy.

Die substituierten Heterocyclen der Formel (II) sind neu und ebenfalls Gegenstand der Erfindung. Man erhält sie, wenn man Cyclohexanonderivate der Formel (V),

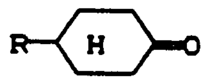

(V)

in welcher

R die oben angegebene Bedeutung hat,

mit Alkoholen der Formel (VI),

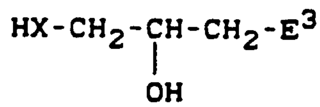

(VI)

in welcher

X die oben angegebene Bedeutung hat und

$E^3$ für Halogen oder Hydroxy steht,

gegebenenfalls in Gegenwart eines Verdünnungsmittels, wie beispielsweise Toluol, und gegebenenfalls in Gegenwart eines sauren Katalysators, wie beispielsweise p-Toluolsulfonsäure, bei Temperaturen zwischen 40 °C und 150 °C cyclisiert und gegebenenfalls in den Fällen, wo $E^3$ in Formel (VI) für eine Hydroxygruppe steht, in einer 2. Stufe die so erhältlichen Hydroxymethylheterocyclen der Formel (VII),

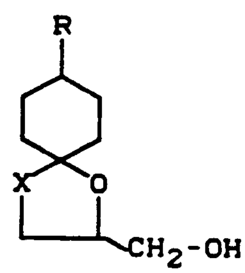

(VII)

in welcher

X und R die oben angegebene Bedeutung haben,

mit gegebenenfalls substituierten Alkyl- oder Arylsulfonylhalogeniden der Formel (VIII),

$Z\text{-}SO_2\text{-}Hal$ (VIII)

in welcher

Hal für Halogen, insbesondere für Chlor steht und

Z für jeweils gegebenenfalls durch Halogen, wie Fluor, Chlor, Brom oder Iod substituiertes Alkyl oder für gegebenenfalls durch Alkyl mit 1 bis 4 Kohlenstoffatomen substituiertes Aryl, wie insbesondere Methyl, Trifluormethyl oder 4-Methylphenyl steht,

gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin, bei Temperaturen zwischen - 20 °C und + 100 °C umsetzt.

Die dabei erhältlichen geometrischen Isomeren lassen sich entweder als Gemische im erfindungsgemäßen Verfahren (a) weiter umsetzen oder mit üblichen Trennmethoden (Chromatographie, Kristallisation) auftrennen.

Die Cyclohexanon-Derivate der Formel (V) sind bekannt oder lassen sich in Analogie zu bekannten Verfahren herstellen (vergl. z.B. Tetrahedron Letters 28, 2347-2350 [1987]; Tetrahedron Lett. 27, 2875-2878 [1986]; Tetrahedron Lett. 1979, 3209-3212; J. Am. chem. Soc. 109, 6887-6889 [1987]; J. Am. chem. Soc. 95, 3646-3651 [1973]; J. Am. chem. Soc. 94, 7599-7600 [1972]; Bull. chem. Soc. Jap. 60, 1721-1726 [1987]; Chem. Lett. 1986, 1593-1596; Synth. Commun. 15, 759-764 [1985]; Synth. Commun. 12, 267-277 [1982]; J. chem. Soc. chem. Commun. 1984, 762-763; J. org. Chem. 38, 1775-1776 [1973]; US 4 251 398; US 3 960 961; EP 2136; DE 26 36 684; DE 25 09 183; FR 22 31 650 sowie die Herstellungsbeispiele).

Die Alkohole der Formel (VI) und die Sulfonylhalogenide der Formel (VIII) sind allgemein bekannte Verbindungen der organischen Chemie.

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten Aminomethylheterocyclen sind durch die Formel (Ia) allgemein definiert. In dieser Formel (Ia) stehen X, R und $R^1$ vorzugsweise für diejenigen Reste, die bereits im Zusammenhang mit der Beschreibung der erfindungsgemäßen Stoffe der Formel (I) für diese Substituenten genannt wurden.

Die Aminomethylheterocyclen der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil, für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffstomen im geradkettigen oder verzweigten Alkyl- bzw. Alkenylteil, wobei als Arylsubstituenten jeweils infrage kommmen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen.

$R^{2-1}$ steht besonders bevorzugt für Methyl, Ethyl, n-oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Dioxolanylmethyl, Dioxolanylethyl, Dioxanylmethyl, Dioxanylethyl, Oxolanylmethyl, Oxolanylethyl, für gegebenenfalls jeweils ein- bis fünffach, gleich oder verschieden durch Fluor, Chlor, Brom, Methyl, Ethyl, n- oder i-Propyl, n-, i-, s- und/oder t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Benzyl oder Phenylethyl, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

$R^{2-1}$ steht ganz besonders bevorzugt für Methyl, Ethyl, n- oder i-Propyl, n-, i-, s- oder t-Butyl, n- oder

i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Cyclopropylmethyl, Oxolanylmethyl, Oxolanylethyl, Dichlorcyclopropylmethyl, Dimethylcyclopropylmethyl, Dichlordimethylcyclopropylmethyl, Cyclopentyl, Cylohexyl oder Cyclohexylmethyl.

$E^2$ steht vorzugsweise für Halogen, insbesondere für Chlor, Brom oder Iod oder für jeweils gegebenenfalls durch Halogen, wie Fluor, Chlor, Brom oder Iod substituiertes Alkylsulfonyloxy oder Alkoxysulfonyloxy mit jeweils 1 bis 4 Kohlenstoffatomen oder für gegebenenfalls durch z.B. Alkyl mit 1 bis 4 Kohlenstoffatomen substituiertes Arylsulfonyloxy, wie beispielsweise Methansulfonyloxy, Methoxysulfonyloxy oder p-Toluolsulfonyloxy.

Die Alkylierungsmittel der Formel (IV) sind ebenfalls allgemein bekannte Verbindungen der organischen Chemie oder erhältlich in Analogie zu allgemein bekannten Verfahren.

Als Verdünnungsmittel zur Durchführung der erfindungsgemäßen Verfahren (a) und (b) kommen inerte organische Lösungsmittel oder wäßrige Systeme infrage.
Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid oder Alkohole, wie Methanol, Ethanol oder Propanol.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid oder Trimethylbenzylammoniumchlorid. Es ist auch möglich, die erfindungsgemäßen Verfahren (a) und (b) ohne Zusatz eines Lösungsmittels durchzuführen.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydroxide, -alkoholate, -carbonate oder -hydrogencarbonate, wie beispielsweise Natriumhydroxid, Natriummethylat, Na-triumethylat, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (III) bzw. (Ia) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbeitet man bei Temperaturen zwischen + 20 °C und + 200 °C, vorzugsweise bei Temperaturen zwischen 80 °C und +180 °C.

Die erfindungsgemäßen Verfahren (a) und (b) werden im allgemeinen bei Normaldruck durchgeführt. Es ist jedoch auch möglich unter erhöhtem Druck im Bereich zwischen 1 und 10 atm zu arbeiten. Die Arbeitsweise unter erhöhtem Druck empfiehlt sich insbesondere, wenn ein oder mehrere Reaktionsteilnehmer bei Normaldruck und der erforderlichen Reaktionstemperatur gasförmig vorliegen.

Zur Durchführung des erfindungsgemäßen Verfahrens (a) setzt man pro Mol an substituiertem Heterocyclus der Formel (II) im allgemeinen 1,0 bis 10,0 Mol, vorzugsweise 1,0 bis 5,0 Mol an Amin der Formel (III) und gegebenenfalls 1,0 bis 10,0 Mol, vorzugsweise 1,0 bis 5,0 Mol an Säurebindemittel, und gegebenenfalls 0,1 bis 1,0 Mol an Phasentransferkatalysator ein.

Zur Durchführung des erfindungsgemäßen Verfahrens (b) setzt man pro Mol an Aminomethylheterocyclus der Formel (Ia) im allgemeinen 1,0 bis 5,0 Mol, vorzugsweise 1,0, bis 2,0 Mol an Alkylierungsmittel der Formel (IV) und 1,0 bis 5,0 Mol, vorzugsweise 1,0 bis 2,0 Mol an Säurebindemittel, und gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Die pflanzenverträglichen Säureadditionssalze der Verbindungen der Formel (I) können in einfacher

Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunspelzigkeit des Weizens (Leptosphaeria nodorum) oder gegen den Erreger der Blattfleckenkrankheit der Gerste (Pyrenophora teres) oder gegen den Erreger der Blattfleckenkrankheit des Weizens (Cochliobolus sativus) sowie gegen Mehltau und Rostarten oder zur Bekämpfung von Krankheiten im Obst- und Gemüseanbau, wie beispielsweise gegen den Erreger des Apfelschorfes (Venturia inaequalis), gegen Phytophthora- und Botrytis-Arten oder zur Bekämpfung von Krankheiten im Reisanbau, wie beispielsweise gegen den Erreger der Reisfleckenkrankheit (Pyricularia oryzae) einsetzen.

Darüberhinaus zeigen die erfindungsgemäßen Wirkstoffe eine gute insektizide Wirksamkeit beispielsweise gegen Blattläuse (Myzus persicae) sowie pflanzenwachstumsregulierende Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln

und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können auch im Materialschutz zum Schutz technischer Materialien eingesetzt werden.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise

wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeroginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, Vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt:
Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol.

<u>Herstellungsbeispiele</u>

**Beispiel 1:**

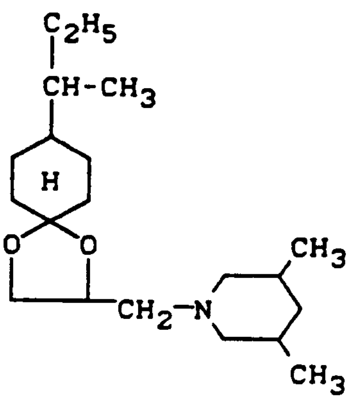

(Verfahren a)

24,7 g (0,1 Mol) 8-s-Butyl-2-chlormethyl-1,4-dioxaspiro-[4,5]decan und 45,2 g (0,4 Mol) cis-3,5-Dimethylpiperidin werden 16 Stunden bei 140 °C gerührt, abgekühlt, mit 200 ml Essigester versetzt, dreimal mit jeweils 200 ml Wasser gewaschen, über Natriumsulfat getrocknet, im Vakuum eingeengt und der Rückstand chromatographisch (Kieselgel; Laufmittel: Essigester) gereinigt.

Man erhält 24,3 g (75 % der Theorie) an 8-s-Butyl-2-(3,5-dimethylpiperidin-1-ylmethyl)-1,4-dioxaspiro-[4,5]decan als Öl vom Brechungsindex $n_D^{20}$ 1,4762.

Herstellung der Ausgangsverbindungen:

**Beispiel II-1:**

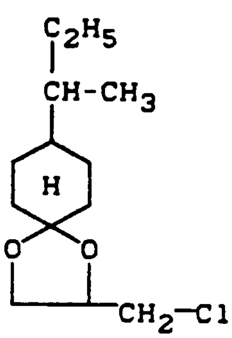

77 g (0,5 Mol) 4-s-Butylcyclohexanon in 1000 ml Toluol werden mit 83,7 ml (1 Mol) 3-Chlorpropan-1,2-diol und 9,5 g (0,05 Mol) p-Toluolsulfonsäure versetzt, 16 Stunden über einem Wasserabscheider unter Rückfluß erhitzt, abgekühlt, dreimal mit jeweils 1000 ml gesättigter wässriger Natriumhydrogencarbonatlösung gewaschen, über Natriumsulfat getrocknet, im Vakuum eingeengt und im Hochvakuum getrocknet.

Man erhält 120,4 g (98 % der Theorie) an 8-s-Butyl-2-chlormethyl-1,4-dioxaspiro[4,5]decan als Öl vom Brechungsindex $n_D^{20}$ 1,4776.

**Beispiel V-a:**

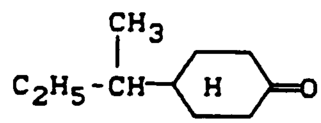

Zu einer Lösung von 106,6 g (0,4 Mol) Natriumdichromat in 812 ml Wasser gibt man 156 g (1 Mol) 4-s-Butyl-cyclohexanol, tropft anschließend bei 70 °C 92,9 ml konzentrierte Schwefelsäure zu, läßt abkühlen auf Raumtemperatur, extrahiert viermal mit jeweils 500 ml Essigester, wäscht die vereinigten organischen Phasen zweimal mit jeweils 500 ml 5 %iger Natronlauge und einmal mit 500 ml Wasser, trocknet über Natriumsulfat und entfernt anschließend das Lösungsmittel im Vakuum.

Man erhält 131,3 g (85 % der Theorie) an 4-s-Butylcyclohexanon vom Brechnungsindex $n_D^{20}$ 1,3547.

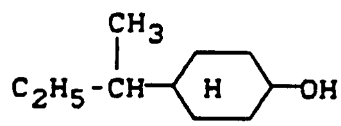

Zu 200 g (1,33 Mol) 4-s-Butylphenol (vergl. z.B. US 3 418 379) in 200 ml Isopropanol gibt man 20 g Ruthenium (5 % auf Aktivkohle) und hydriert bei 150 °C und 200 bar Wasserstoffdruck im Autoklaven. Zur Aufarbeitung wird der Katalysator abfiltriert und das Lösungsmittel abdestilliert.

Man erhält 193,4 g (93 % der Theorie) an 4-s-Butylcyclohexanol als Öl vom Brechungsindex $n_D^{20}$ 1,4660.

In entsprechender Weise und gemäß den allgemeinen Angaben zur Herstellung erhält man die folgenden Aminomethylheterocyclen der allgemeinen Formel (I):

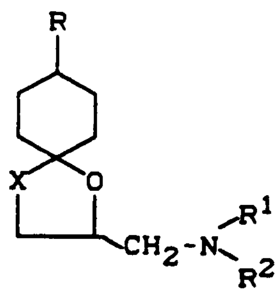

(I)

| Bsp. Nr. | X | R | 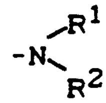 | physikalische Eigenschaften |
|---|---|---|---|---|
| 2 | O | 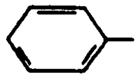 | 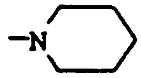 | $n_D^{20}$ 1,5275 |
| 3 | O | 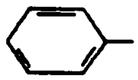 | 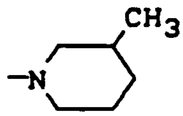 | $n_D^{20}$ 1,5214 |
| 4 | O | 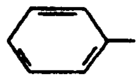 | 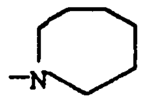 | $n_D^{20}$ 1,5289 |
| 5 | O | 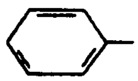 | 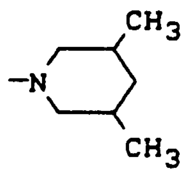 | $n_D^{20}$ 1,5165 |
| 6 | O | 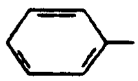 | 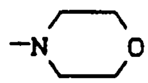 | $n_D^{20}$ 1,5304 |
| 7 | O | 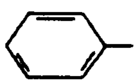 | 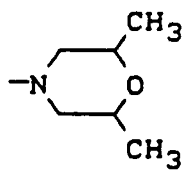 | $n_D^{20}$ 1,5181 |
| 8 | O | 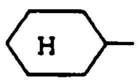 | 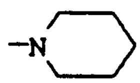 | $n_D^{20}$ 1,4974 |
| 9 | O | 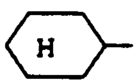 | 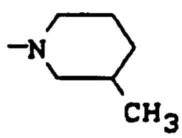 | $n_D^{20}$ 1,4935 |
| 10 | O | 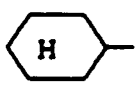 | 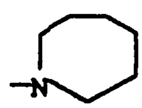 | $n_D^{20}$ 1,4997 |
| 11 | O | 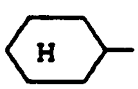 | 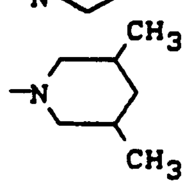 | $n_D^{20}$ 1,4909 |

| Bsp. Nr. | X | R | 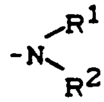 | physikalische Eigenschaften |
|---|---|---|---|---|
| 12 | O | 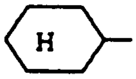 | 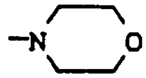 | $n_D^{20}$ 1,4972 |
| 13 | O | 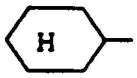 | 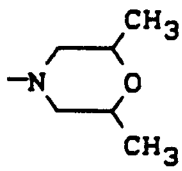 | $n_D^{20}$ 1,4901 |
| 14 | O | 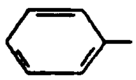 | 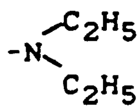 | $n_D^{20}$ 1,5113 |
| 15 | O | 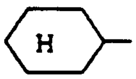 | 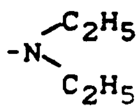 | $n_D^{20}$ 1,4828 |
| 16 | O | 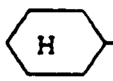$-CH_2-$ | 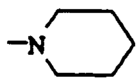 | $n_D^{20}$ 1,4969 |
| 17 | O | 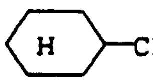$-CH_2-$ | 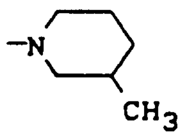 | $n_D^{20}$ 1,4929 |
| 18 | O | 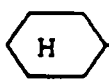$-CH_2-$ | 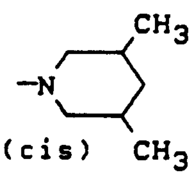 (cis) | $n_D^{20}$ 1,4841 |
| 19 | O | 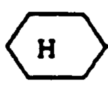$-CH_2-$ | 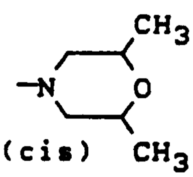 (cis) | $n_D^{20}$ 1,4868 |
| 20 | O | 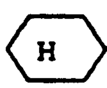$-CH_2-$ | 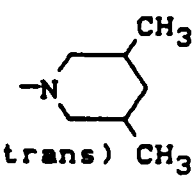 (trans) | $n_D^{20}$ 1,4925 |

| Bsp. Nr. | X | R | 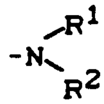 | physikalische Eigenschaften |
|---|---|---|---|---|
| 21 | O | $H_3C-(CH_2)_2-$ | 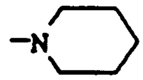 | $n_D^{20}$ 1,4790 |
| 22 | O | $H_3C-(CH_2)_2-$ | 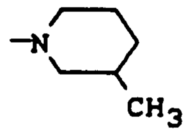 | $n_D^{20}$ 1,4764 |
| 23 | O | $H_3C-(CH_2)_2-$ | 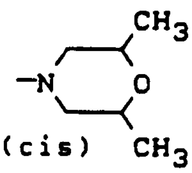 (cis) | $n_D^{20}$ 1,4729 |
| 24 | O | $H_3C-(CH_2)_2-$ | 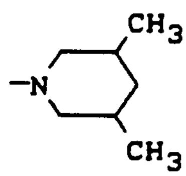 | $n_D^{20}$ 1,4746 |
| 25 | O | $H_3C-(CH_2)_2-$ | 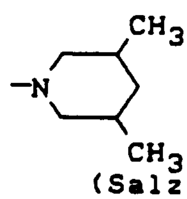 | $^1$H-NMR*): 3,5-3,68(m,3H); 4,1-4,3 (m,1H); 4,6-4,75(m,1H) (Salz mit Saccharin) |
| 26 | O | $H_3C-(CH_2)_2-$ | 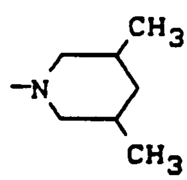 | $^1$H-NMR*): 3,4-3,9 (m,3H); 4,05-4,2(m,1H); 4,6-4,75(m,1H) (Salz mit p-Dodecylbenzolsulfonsäure) |
| 27 | O | 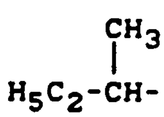 | 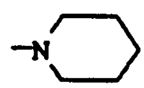 | $n_D^{20}$ 1,4816 |
| 28 | O | 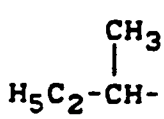 | 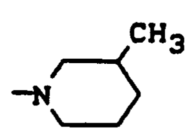 | $n_D^{20}$ 1,4795 |

| Bsp. Nr. | X | R | 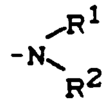 | physikalische Eigenschaften |
|---|---|---|---|---|
| 29 | O | 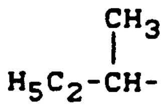 | 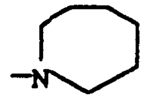 | $n_D^{20}$ 1,4866 |
| 30 | O | 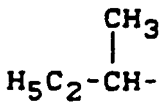 | 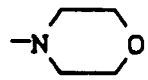 | $n_D^{20}$ 1,4826 |
| 31 | O | 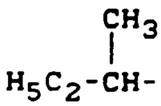 | 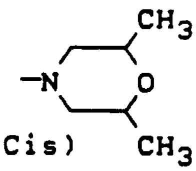 (Cis) | $n_D^{20}$ 1,4775 |
| 32 | O | 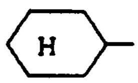 | $-NH-C_2H_5$ | $n_D^{20}$ 1,4869 |
| 33 | O | 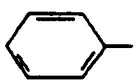 | 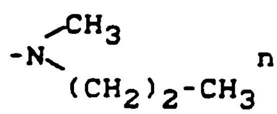 | $n_D^{20}$ 1,5110 |
| 34 | O | 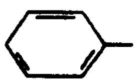 | 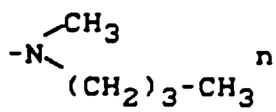 | $n_D^{20}$ 1,5085 |
| 35 | O | 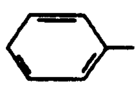 | 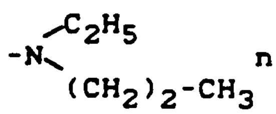 | $n_D^{20}$ 1,5086 |
| 36 | O | 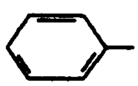 | 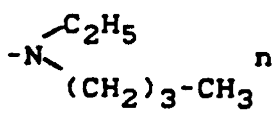 | $n_D^{20}$ 1,5067 |
| 37 | O | 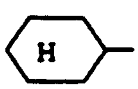 | 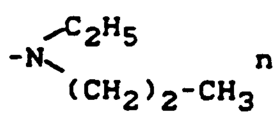 | $n_D^{20}$ 1,4821 |
| 38 | O | 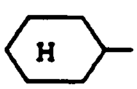 | 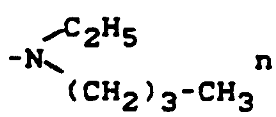 | $n_D^{20}$ 1,4825 |

| Bsp. Nr. | X | R | 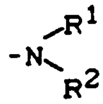 | physikalische Eigenschaften |
|---|---|---|---|---|
| 39 | O | 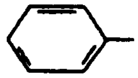 | 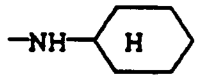 | $n_D^{20}$ 1,5254 |
| 40 | O | 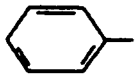 | 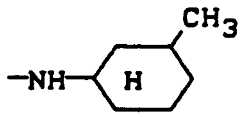 | $n_D^{20}$ 1,5211 |
| 41 | O | 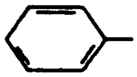 | 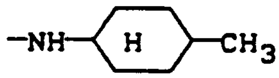 | $n_D^{20}$ 1,5174 |
| 42 | O | 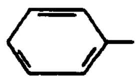 | 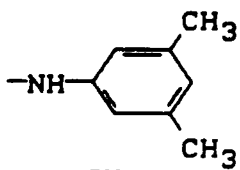 | $^1$H-NMR*): 3,1-3,4 (m,2H); 3,75-3,85(m,2H) |
| 43 | O | 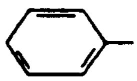 | 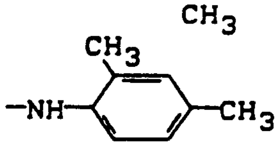 | $^1$H-NMR*): 3,1-3,4(m,2H); 3,7-3,9(m,2H); |
| 44 | O | 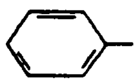 | 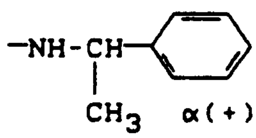 α(+) | $^1$H-NMR*): 3,5-3,9(m,2H) 3,95-4,05(m,1H) |
| 45 | O | 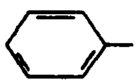 | 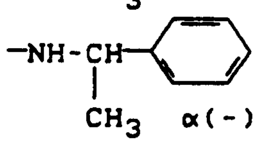 α(-) | $^1$H-NMR*): 3,5-3,9(m,2H); 4,0-4,1(m,1H) |
| 46 | O | 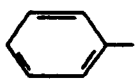 | $-NH-(CH_2)_3-CH_3$ | $n_D^{20}$ 1,5145 |
| 47 | O | 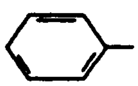 | $-N(CH_2)_2-CH_3$ / $(CH_2)_3-CH_3$ | $n_D^{20}$ 1,5049 |
| 48 | O | 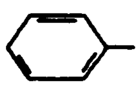 | $-N(CH_2)_3-CH_3$ / $(CH_2)_3-CH_3$ | $n_D^{20}$ 1,5031 |

| Bsp. Nr. | X | R | 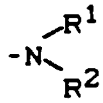 | physikalische Eigenschaften |
|---|---|---|---|---|
| 49 | O | 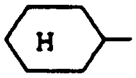 | 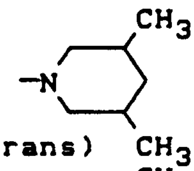 (trans) | $^{1}H$-NMR*): 3,4-3,7(m,2H); 4,0-4,15(m,1H) |
| 50 | O | 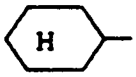 | 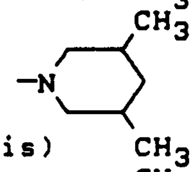 (cis) | $^{1}H$-NMR*): 3,5-3,6(m,2H); 4,0-4,1(m,1H) (Diastereomeres A) |
| 51 | O | 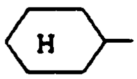 | 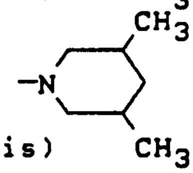 (cis) | $^{1}H$-NMR*): 3,55-3,65(m,2H); 4,0-4,1(m,1H) (Diastereomeres B) |
| 52 | O | 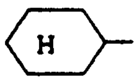 | 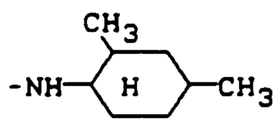 | $^{1}H$-NMR*): 3,4-3,8(m,2H); 3,95-4,1(m,1H) |
| 53 | O | 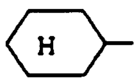 | 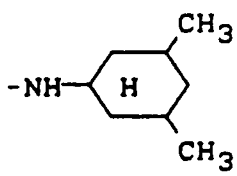 | $^{1}H$-NMR*): 3,6-3,8(m,1H) 3,9-4,15(m,2H) |
| 54 | O | 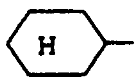 | 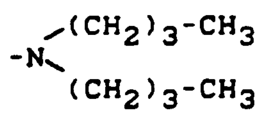 | $^{1}H$-NMR*): 3,4-3,7(m,2H); 3,95-4,1(m,1H) |
| 55 | O | 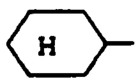 | 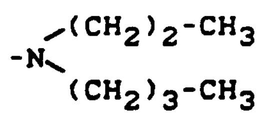 | $^{1}H$-NMR*): 3,45-3,7(m,2H); 3,95-4,1(m,1H) |
| 56 | O | 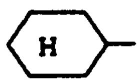 | 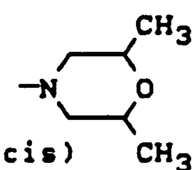 (cis) | $n_D^{20}$ 1,4909 (Diastereomeres A) |
| 57 | O | 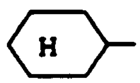 | 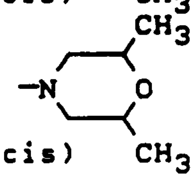 (cis) | $n_D^{20}$ 1,4884 (Diastereomeres B) |

| Bsp. Nr. | X | R | 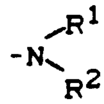 | physikalische Eigenschaften |
|---|---|---|---|---|
| 58 | O | 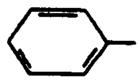 | 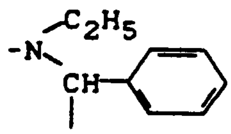 α(+) | $n_D^{20}$ 1,5370 |
| 59 | O | 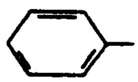 | 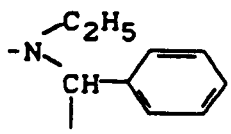 α(-) | $n_D^{20}$ 1,5413 |
| 60 | O | 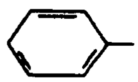 | 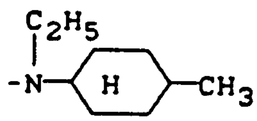 | $^1H$-NMR*): 3,65-3,75(m,1H); 4,0-4,2(m,2H) |
| 61 | O | 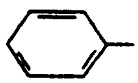 | 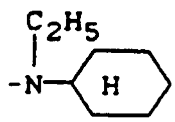 | $^1H$-NMR*): 3,65-3,8(m,1H); 4,0-4,25(m,2H) |
| 62 | O | 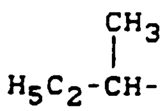 | $-NH-(CH_2)_2-CH_3$ | Kp 120° C/ 0,5 mbar |
| 63 | O | 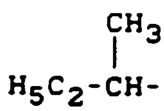 | $-NH-(CH_2)_2-OCH_3$ | Kp 125° C/ 0,5 mbar |
| 64 | O | 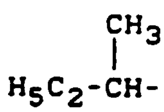 | 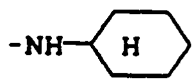 | $n_D^{20}$ 1,4837 |
| 65 | O | 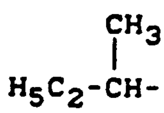 | 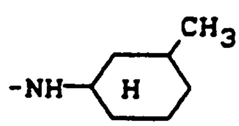 | $n_D^{20}$ 1,4805 |
| 66 | O | 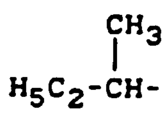 | 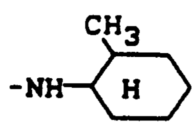 | $n_D^{20}$ 1,4851 |
| 67 | O | 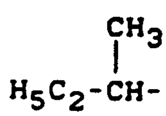 | 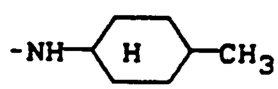 | $n_D^{20}$ 1,4793 |

| Bsp. Nr. | X | R | 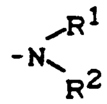 | physikalische Eigenschaften |
|---|---|---|---|---|
| 68 | O | 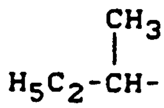 | 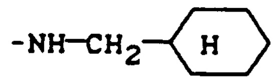 | $n_D^{20}$ 1,4797 |
| 69 | S | 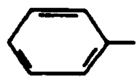 | 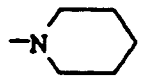 | Fp: 29-31° C |
| 70 | S | 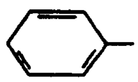 | 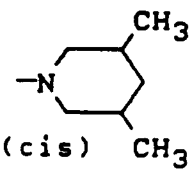 (cis) | Fp: 34-36° C |
| 71 | S | 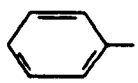 | 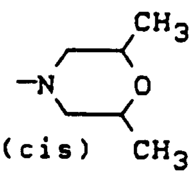 (cis) | Fp: 38° C |
| 72 | S | 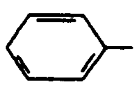 | 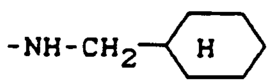 | $n_D^{20}$ 1,5462 |
| 73 | S | 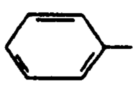 | 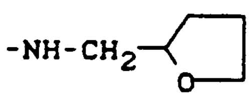 | $n_D^{20}$ 1,5514 |
| 74 | S | 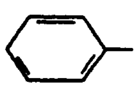 | 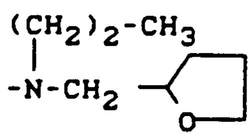 | $n_D^{20}$ 1,5364 |
| 75 | S | 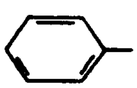 | $-NH-(CH_2)_3-OC_2H_5$ | $n_D^{20}$ 1,5329 |
| 76 | S | 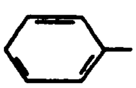 | 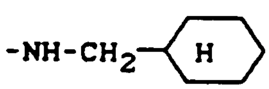 | $n_D^{20}$ 1,5591 (Salz mit Saccharin) |

| Bsp. Nr. | X | R | 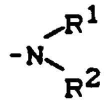 | physikalische Eigenschaften |
|---|---|---|---|---|
| 77 | S | 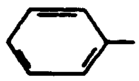 | 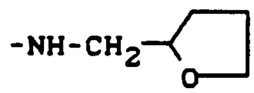 | $n_D^{20}$ 1,5600 (Salz mit Saccharin) |
| 78 | S | 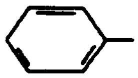 | 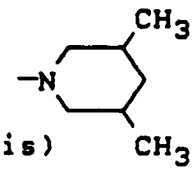 (cis) | $n_D^{20}$ 1,5452 (Salz mit Saccharin) |
| 79 | S | 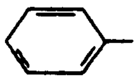 | 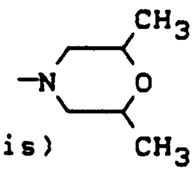 (cis) | $n_D^{20}$ 1,5509 (Salz mit Saccharin) |
| 80 | S | 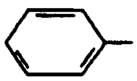 | 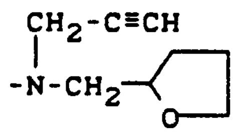 | $n_D^{20}$ 1,5539 |
| 81 | S | 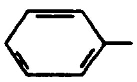 | 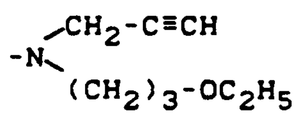 | $n_D^{20}$ 1,5440 |
| 82 | S | 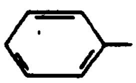 | 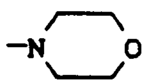 | $n_D^{20}$ 1,5538 |
| 83 | S | 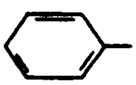 | 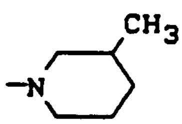 | $^1$H-NMR*): 4,3-4,55(m,1H) 3,0-3,2 (m,2H) |
| 84 | S | 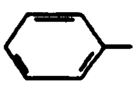 | 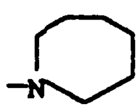 | 4,25-4,5(m,1H) 3,05-3,15(m,1H) 2,6-3,0 (m,7H) |
| 85 | S | 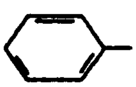 | 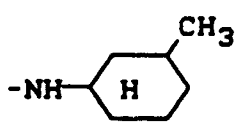 | $n_D^{20}$ 1,5346 |

| Bsp. Nr. | X | R | $-N(R^1)(R^2)$ | physikalische Eigenschaften |
|---|---|---|---|---|
| 86 | S | 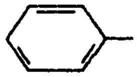 | $-NH-CH_2-CH(CH_3)_2$ | $n_D^{20}$ 1,5269 |
| 87 | S | 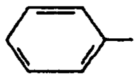 | $-NH-CH_2-CH(C_2H_5)_2$ | $n_D^{20}$ 1,5074 |
| 88 | S | 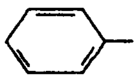 | 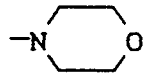 | $^1$H-NMR*): 4,65-4,9(m,1H) 3,9-4,15(m,4H) 3,05-3,65(m,4H) (Salz mit Saccharin) |
| 89 | S | 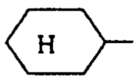 | 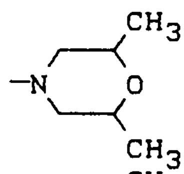 | $n_D^{20}$ 1,5141 |
| 90 | S | 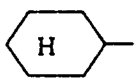 | 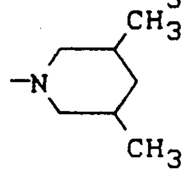 | $n_D^{20}$ 1,4990 |
| 91 | S | 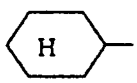 | -NH- 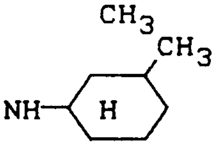 | $n_D^{20}$ 1,5232 |
| 92 | S | 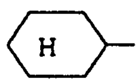 | $-NH-(CH_2)_2-OCH_3$ | $n_D^{20}$ 1,5948 |
| 93 | S | 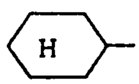 | $-N((CH_2)_2-CH_3)((CH_2)_2-OCH_3)$ | $n_D^{20}$ 1,5068 |

| Bsp. Nr. | X | R | 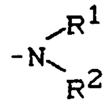 | Physikalische Eigenschaften |
|---|---|---|---|---|
| 94 | O | $-CH(CH_3)C_2H_5$ | 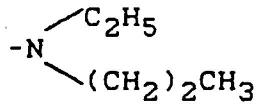 | $n_D^{20}$ 1.4635 |
| 95 | O | $-CH(CH_3)C_2H_5$ | 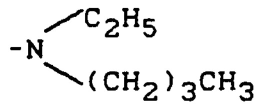 | $n_D^{20}$ 1.4640 |
| 96 | O | $-CH(CH_3)C_2H_5$ | 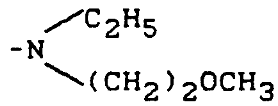 | $n_D^{20}$ 1.4640 |
| 97 | O | $-CH(CH_3)C_2H_5$ | 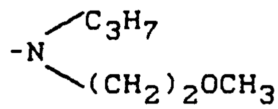 | $n_D^{20}$ 1.4619 |
| 98 | O | $-CH(CH_3)C_2H_5$ | 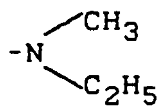 | $n_D^{20}$ 1.4633 |
| 99 | O | $-CH(CH_3)C_2H_5$ | 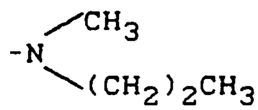 | $n_D^{20}$ 1.4629 |
| 100 | O | $-CH(CH_3)C_2H_5$ | 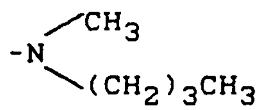 | $n_D^{20}$ 1.4635 |
| 101 | O | $-CH(CH_3)C_2H_5$ | 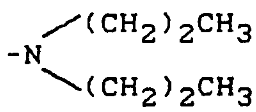 | $n_D^{20}$ 1.4645 |
| 102 | O | $-CH(CH_3)C_2H_5$ | 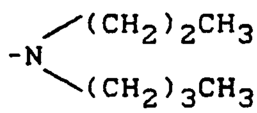 | $n_D^{20}$ 1.4637 |
| 103 | O | 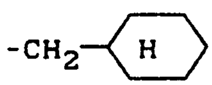 | 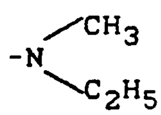 | $n_D^{20}$ 1.4794 |
| 104 | O | 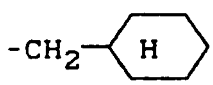 | 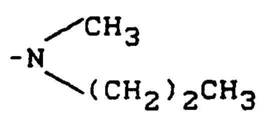 | $n_D^{20}$ 1.4789 |

| Bsp. Nr. | X | R | 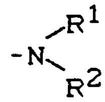 | Physikalische Eigenschaften |
|---|---|---|---|---|
| 105 | O | 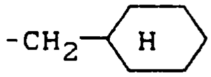 | 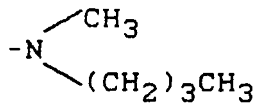 | $n_D^{20}$ 1.4775 |
| 106 | O | 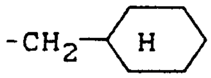 | 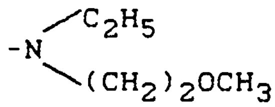 | $n_D^{20}$ 1.4772 |
| 107 | O | 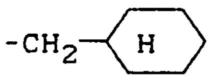 | 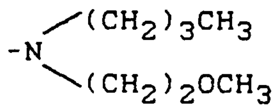 | $n_D^{20}$ 1.4757 |
| 108 | O | $-CH(CH_3)C_3H_7$ | 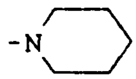 | $n_D^{20}$ 1.4816 |
| 109 | O | $-CH(CH_3)C_3H_7$ | 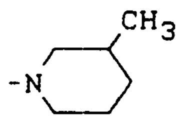 | $n_D^{20}$ 1.4821 |
| 110 | O | $-CH(CH_3)C_3H_7$ | 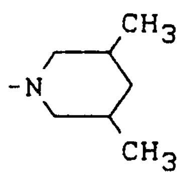 | $n_D^{20}$ 1.4782 |
| 111 | O | $-CH(CH_3)C_3H_7$ | 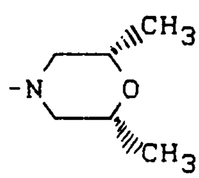 | $n_D^{20}$ 1.4757 |
| 112 | O | $-CH(C_2H_5)C_2H_5$ | 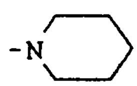 | $n_D^{20}$ 1.4832 |
| 113 | O | $-CH(C_2H_5)C_2H_5$ | 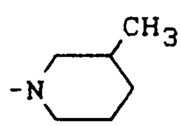 | $n_D^{20}$ 1.4810 |

| Bsp. Nr. | X | R | 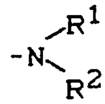 | Physikalische Eigenschaften |
|---|---|---|---|---|
| 114 | O | $-CH(C_2H_5)C_2H_5$ | 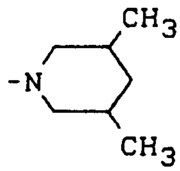 | $n_D^{20}$ 1.4792 |
| 115 | O | $-CH(C_2H_5)C_2H_5$ | 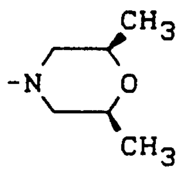 | $n_D^{20}$ 1.4766 |
| 116 | O | $-CH(C_2H_5)C_2H_5$ | -NH 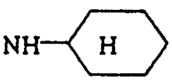 | $n_D^{20}$ 1.4863 |
| 117 | O | $-CH(C_2H_5)C_2H_5$ | $-N(CH_3)$ 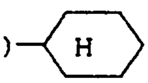 | $n_D^{20}$ 1.4857 |
| 118 | O | $-CH(C_2H_5)C_2H_5$ | -NH 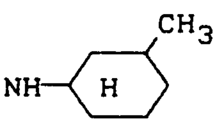 | $n_D^{20}$ 1.4825 |
| 119 | O | $-CH(CH_3)CH_2CH(CH_3)_2$ | 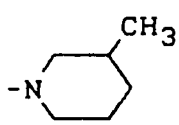 | $n_D^{20}$ 1.4799 |
| 120 | O | $-CH(CH_3)CH_2CH(CH_3)_2$ | 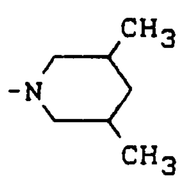 | $n_D^{20}$ 1.4769 |

| Bsp. Nr. | X | R | 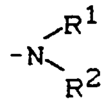 | Physikalische Eigenschaften |
|---|---|---|---|---|
| 121 | O | $-CH(CH_3)C_5H_{11}$ | $-N(CH_3)-$ 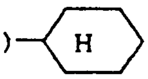 | $n_D^{20}$ 1,3890 |
| 122 | O | $-CH(CH_3)C_5H_{11}$ | 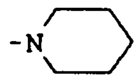 | $n_D^{20}$ 1.4808 |
| 123 | O | $-CH(CH_3)C_5H_{11}$ | 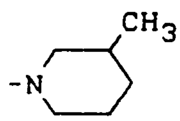 | $n_D^{20}$ 1.4805 |
| 124 | O | $-CH(C_2H_5)C_2H_5$ | $-NH-(CH_2)_2OCH_3$ | $n_D^{20}$ 1.4707 |
| 125 | O | $-CH(C_2H_5)C_2H_5$ | $-NH-(CH_2)_2CH_3$ | $n_D^{20}$ 1,4687 |
| 126 | O | $-CH(CH_3)CH_2CH(CH_3)_2$ | $-NH-(CH_2)_2CH_3$ | $n_D^{20}$ 1.4659 |
| 127 | O | $-CH(CH_3)C_5H_{11}$ | 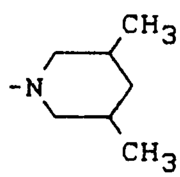 | $n_D^{20}$ 1.4767 |
| 128 | O | $-CH(CH_3)C_5H_{11}$ | 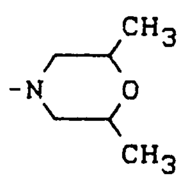 | $n_D^{20}$ 1.4759 |
| 129 | O | $-CH(CH_3)C_5H_{11}$ | -NH- 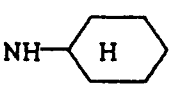 | $n_D^{20}$ 1,4855 |
| 130 | O | $-CH(CH_3)C_5H_{11}$ | -NH- 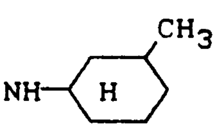 | $n_D^{20}$ 1.4813 |

| Bsp. Nr. | X | R | 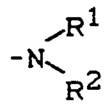 | Physikalische Eigenschaften |
|---|---|---|---|---|
| 131 | O | $-CH(CH_3)C_5H_{11}$ | $-NH-C_2H_5$ | $n_D^{20}$ 1,4525 |
| 132 | O | $-CH(CH_3)C_5H_{11}$ | $-NH-(CH_2)_2CH_3$ | $n_D^{20}$ 1.4669 |
| 133 | O | $-CH(C_2H_5)C_2H_5$ | 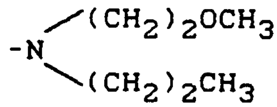 | $n_D^{20}$ 1.4623 |
| 134 | O | $-CH(C_2H_5)C_2H_5$ | 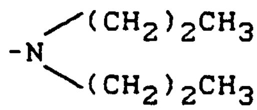 | $n_D^{20}$ 1.4636 |
| 135 | O | $-CH(CH_3)CH_2CH(CH_3)_2$ | 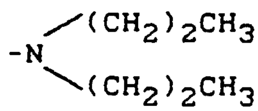 | $n_D^{20}$ 1.4642 |
| 136 | O | $-CH(C_2H_5)C_2H_5$ | 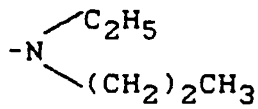 | $n_D^{20}$ 1.4675 |
| 137 | O | $-CH(C_2H_5)C_2H_5$ | 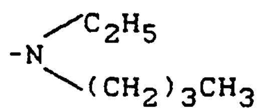 | $n_D^{20}$ 1.4670 |
| 138 | O | $-CH(CH_3)C_3H_7$ | 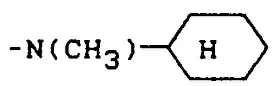 | $n_D^{20}$ 1.4869 |
| 139 | O | $-CH(CH_3)C_3H_7$ | 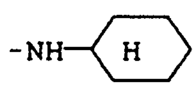 | $n_D^{20}$ 1.4817 |
| 140 | O | $-CH(CH_3)C_3H_7$ | 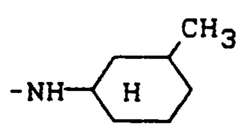 | $n_D^{20}$ 1.4834 |
| 141 | O | $-CH(CH_3)C_3H_7$ | $-NH-C_2H_5$ | $n_D^{20}$ 1,4657 |

| Bsp. Nr. | X | R | 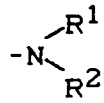 | Physikalische Eigenschaften |
|---|---|---|---|---|
| 142 | O | $-CH(CH_3)C_3H_7$ | $-NH-(CH_2)_2OCH_3$ | $n_D^{20}$ 1.4677 |
| 143 | O | $-CH(CH_3)C_3H_7$ | $-NH-(CH_2)_2CH_3$ | $n_D^{20}$ 1.4674 |
| 144 | O | $-CH(CH_3)C_3H_7$ | 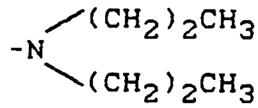 | $n_D^{20}$ 1.4653 |
| 145 | O | $-CH(CH_3)C_3H_7$ | 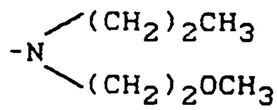 | $n_D^{20}$ 1.4647 |
| 146 | O | $-CH(CH_3)C_3H_7$ | 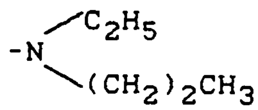 | $n_D^{20}$ 1.4633 |
| 147 | O | $-CH(CH_3)C_5H_{11}$ | 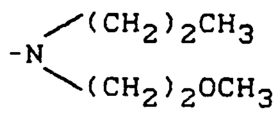 | $n_D^{20}$ 1.4657 |
| 148 | O | $-CH(CH_3)C_3H_7$ | 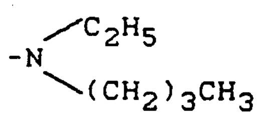 | $n_D^{20}$ 1.4652 |
| 149 | O | $-CH(CH_3)C_5H_{11}$ | 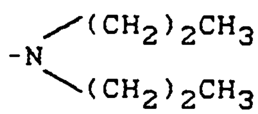 | $n_D^{20}$ 1.4644 |
| 150 | O | $-CH(CH_3)C_5H_{11}$ | 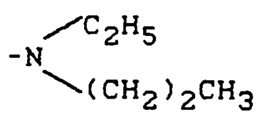 | $n_D^{20}$ 1.4641 |
| 151 | O | $-CH(CH_3)C_5H_{11}$ | 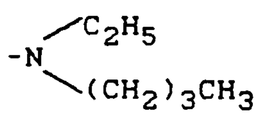 | $n_D^{20}$ 1.4655 |
| 152 | O | 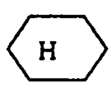 | 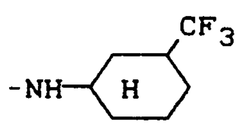 | Öl |

| Bsp. Nr. | X | R | 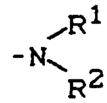 | Physikalische Eigenschaften |
|---|---|---|---|---|
| 153 | O | 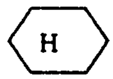 | 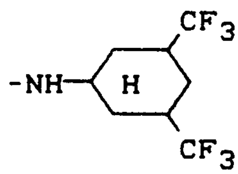 | Öl |
| 154 | O | 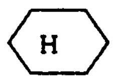 | 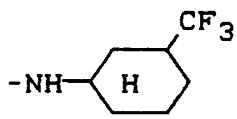 | Öl (Diasterom.A) |
| 155 | O | $-CH(C_3H_7)_2$ | 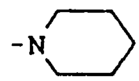 | $n_D^{20}$ 1.4814 |
| 156 | O | $-CH(C_3H_7)_2$ | $-N(CH_3)-$ 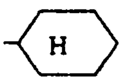 | $n_D^{20}$ 1.4846 |
| 157 | O | $-CH(C_3H_7)_2$ | $-NH-C_3H_7$ | $n_D^{20}$ 1.4664 |
| 158 | O | $-CH(C_3H_7)_2$ | $-NH-(CH_2)_2OCH_3$ | $n_D^{20}$ 1.4659 |
| 159 | O | $-CH(C_2H_5)_2$ | $-NH-C_5H_{11}$ | $n_D^{20}$ 1.4689 |
| 160 | O | $-CH(C_3H_7)_2$ | 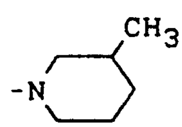 | $n_D^{20}$ 1.4786 |
| 161 | O | $-CH(C_3H_7)_2$ | 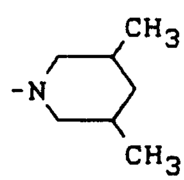 | $n_D^{20}$ 1.4773 |
| 162 | O | $-CH(C_3H_7)_2$ | 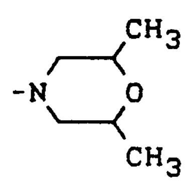 | $n_D^{20}$ 1.4768 |

| Bsp. Nr. | X | R | 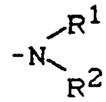 | Physikalische Eigenschaften |
|---|---|---|---|---|
| 163 | O | $-CH(C_3H_7)_2$ | 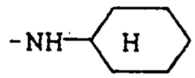 | $n_D^{20}$ 1.4854 |
| 164 | O | $-CH(C_3H_7)_2$ | 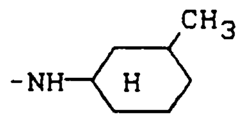 | $n_D^{20}$ 1.4814 |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurden die nachstehend aufgeführten Verbindungen als Vergleichssubstanzen eingesetzt:

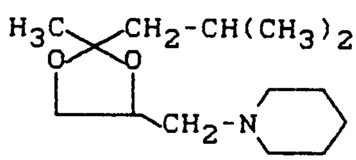

(A)

2-Isobutyl-2-methyl-4-(1-piperidinylmethyl)-1,3-dioxolan

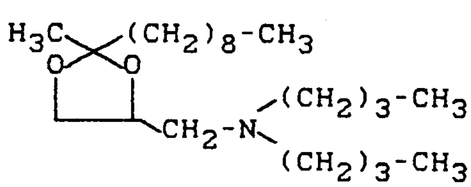

(B)

2-Methyl-2-nonyl-4-di-n-butylaminomethyl-1,3-dioxolan

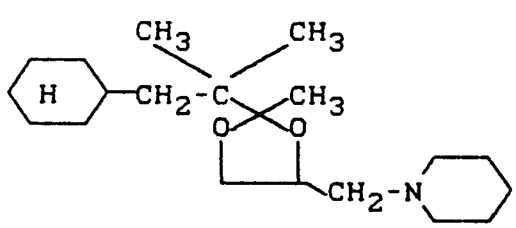

(C)

2-(2-Cyclohexylmethyl-2-propyl)-2-methyl-4-(1-piperidinylmethyl)-1,3-dioxolan

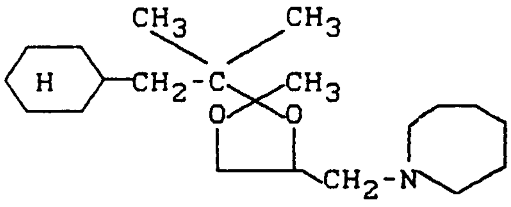

(D)

2-(2-Cyclohexylmethyl-2-propyl)-2-methyl-4-(1-perhydroazepinylmethyl)-1,3-dioxolan (alle bekannt aus EP 97 822).

Beispiel A

Leptosphaeria nodorum-Test (Weizen)/protektiv/
Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.
Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutlich Überlegenheit in der Wirksamkeit gegenüber dem Stand der Technik zeigen bei diesem Test z.B. die Verbindungen gemäß den Herstellungsbeispielen: 3, 8, 9, 10, 14, 26, 27, 28, 29, 39, 40, 41, 44, 45, 46, 47, 48, 52, 55, 70, 72, 75 und 85.

Beispiel B

Cochliobolus sativus-Test (Gerste)/protektiv
Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht, Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 39, 40, 41, 42, 44, 45, 55, 72, 75 und 85.

Beispiel C

Venturia-Test (Apfel) /protektiv
Lösungsmittel: 4,7 Gewichtsteile Aceton
Emulgator: 0,3 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die

gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 40 und 41.

Beispiel D

In einen Agar, der aus Bierwürze und Pepton hergestellt wurde, wurden die erfindungsgemäßen Verbindungen in abgestuften Konzentrationen zwischen 1 und 5000 mg/l Versuchsprobe eingearbeitet. Nach dem Erstarren des Agars erfolgte die Kontamination der so hergestellten Agarproben mit Reinkulturen verschiedener Testpilze.

Nach zweiwöchiger Lagerung bei 28 °C und 60 bis 70 % relativer Luftfeuchtigkeit wurde ausgewertet. In der Tabelle ist als minimale Hemmkonzentration (MHK) die geringste in einer Agarprobe enthaltene Konzentration der Substanz angegeben, bei der keinerlei Bewuchs durch die verwendete Art erfolgte.

In diesem Test zeigen z.B. die Verbindungen 52, 53, 15, 9 und 10 eine sehr gute Wirkung.

**Patentansprüche**

**1.** Aminomethylheterocyclen der allgemeinen Formel (I),

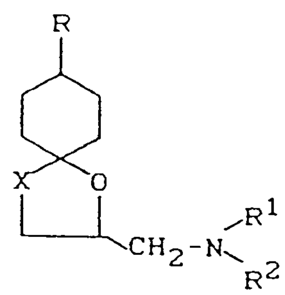

(I)

in welcher

X für Sauerstoff oder Schwefel steht,

R für Cycloalkyl mit 3 bis 7 Kohlenstoffatomen, für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht oder für einen Rest

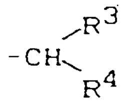

steht,

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 Bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1

bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen,

$R^3$ für Wasserstoff, für geradkettiges oder verzweigtes Alkyl mit 1 Bis 12 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht und

$R^4$ für geradkettiges oder verzweigtes Alkyl mit 1 bis 12 Kohlenstoffatomen, für Cycloalkyl mit 3 bis 7 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht,

wobei jedoch $R^3$ und $R^4$ nicht gleichzeitig für Methyl stehen dürfen, und deren Säureadditionssalze.

**2.** Aminomethylheterocyclen gemäß Anspruch 1, wobei in der Formel (I)

X für Sauerstoff oder Schwefel steht,

R für Cyclopentyl, Cyclohexyl oder für gegebenenfalls ein- bis dreifach, gleich oder verschieden durch Methyl, Ethyl, n- oder i-Propyl, n-, i-, s- oder t-Butyl substituiertes Phenyl steht oder für einen Rest

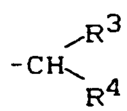

steht,

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls ein- bis fünffach, gleich oder verschieden durch Fluor, Chlor, Brom, Methyl, Ethyl, n- oder i-Propyl, n-, i-, s- oder t-Butyl, Trifluormethyl oder Trifluormethoxy substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen:
Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

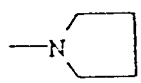 ; 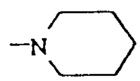 ; 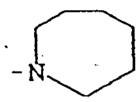 ; 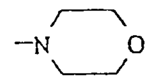

oder 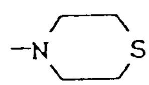

stehen, wobei als Substituenten jeweils infrage kommen: Methyl, Ethyl oder Hydroxymethyl,

und

$R^3$ für Wasserstoff, Methyl, Ethyl, n- oder i-Propyl, für jeweils geradkettiges oder verzweigtes Butyl, Pentyl oder Hexyl steht oder für gegebenenfalls ein- bis dreifach, gleich oder verschieden durch Methyl, Ethyl, n- oder i-Propyl, n-, i-, s- oder t-Butyl substituiertes Phenyl steht und

$R^4$ für Methyl, Ethyl, n- oder i-Propyl, für jeweils geradkettiges oder verzweigtes Butyl, Pentyl oder Hexyl steht, für Cyclopentyl, Cyclohexyl oder für gegebenenfalls ein- bis dreifach, gleich oder verschieden durch Methyl, Ethyl, n- oder i-Propyl, n-, i-, s- oder t-Butyl substituiertes Phenyl steht,

wobei jedoch $R^3$ und $R^4$ nicht gleichzeitig für Methyl stehen dürfen.

**3.** Aminomethylheterocyclen gemäß Anspruch 1, wobei in der Formel (I)

X für Sauerstoff oder Schwefel steht,

R für Cyclohexyl, für Phenyl oder für einen Rest

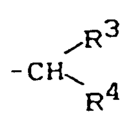

steht,

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

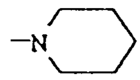 ; 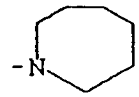 ; 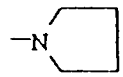 oder 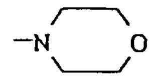

stehen,
wobei als Substituenten jeweils infrage kommen: Methyl, Ethyl oder Hydroxymethyl und

$R^3$ für Wasserstoff, Methyl, Ethyl, n- oder i-Propyl, n-, i-, s- oder t-Butyl oder Phenyl steht und

$R^4$ für Methyl, Ethyl, n- oder i-Propyl, n-, i-, s- oder t-Butyl, für Cyclohexyl oder für Phenyl steht,

wobei jedoch $R^3$ und $R^4$ nicht gleichzeitig für Methyl stehen dürfen.

**4.** Verfahren zur Herstellung von Aminomethylheterocyclen der allgemeinen Formel (I)

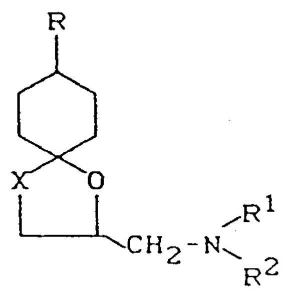

(I)

in welcher

X für Sauerstoff oder Schwefel steht,

R für Cycloalkyl mit 3 bis 7 Kohlenstoffatomen, für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht oder für einen Rest

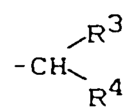

steht,

$R^1$ und $R^2$ unabhängig voneinander jeweils für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten infrage kommen: jeweils geradkettiges oder verzweigtes Alkyl oder Hydroxyalkyl mit jeweils 1 bis 4 Kohlenstoffatomen,

$R^3$ für Wasserstoff, für geradkettiges oder verzweigtes Alkyl mit 1 bis 12 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht und

$R^4$ für geradkettiges oder verzweigtes Alkyl mit 1 bis 12 Kohlenstoffatomen, für Cycloalkyl mit 3 bis 7 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht,

wobei jedoch $R^3$ und $R^4$ nicht gleichzeitig für Methyl stehen dürfen,

und deren Säureadditionssalze, dadurch gekennzeichnet, daß man

(a) substituierte Heterocyclen der Formel (II),

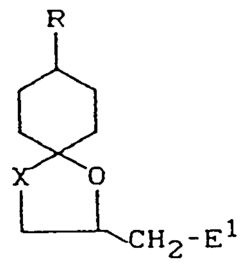

(II)

in welcher

R und X die oben angegebene Bedeutung haben und

$E^1$ für eine elektronenanziehende Abgangsgruppe steht,

mit Aminen der Formel (III),

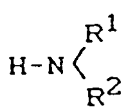

(III)

in welcher

$R^1$ und $R^2$ die oben angegebene Bedeutung haben, gegebenenfalls in Gegenwart eines Verdünnungsmittels und gegebenenfalls in Gegenwart eines Säurebindemittels umsetzt, oder daß man

(b) die nach Verfahren (a) erhältlichen Aminomethylheterocyclen der Formel (Ia),

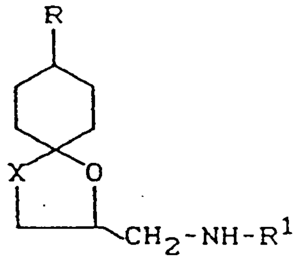

(Ia)

in welcher

R, $R^1$ und X die oben angegebene Bedeutung haben,

mit Alkylierungsmitteln der Formel (IV),

$R^{2-1}$-$E^2$ (IV)

in welcher

$R^{2-1}$ die oben für $R^2$ angegebene Bedeutung, ausgenommen Wasserstoff und Aryl hat und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

**5.** Schädlingsbekämpfungsmittel, gekennzeichnet durch einen Gehalt an mindestens einem Aminomethylheterocyclus der Formel (I) nach den Ansprüchen 1 bis 4.

**6.** Verwendung von Aminomethylheterocyclen der Formel (I) nach den Ansprüchen 1 bis 4 zur Bekämpfung von Schädlingen.

**7.** Verfahren zur Bekämpfung von Schädlingen, dadurch gekennzeichnet, daß man Aminomethylheterocyclen der Formel (I) nach den Ansprüchen 1 bis 4 auf Schädlinge und/oder ihren Lebensraum einwirken läßt.

**8.** Verfahren zur Herstellung von Schädlingsbekämpungsmitteln, dadurch gekennzeichnet, daß man Aminomethylheterocyclen der Formel (I) nach den Ansprüchen 1 bis 4 mit Streckmitteln und/oder oberflächenaktiven Mitteln vermischt.

**9.** Substituierte Heterocyclen der Formel (II),

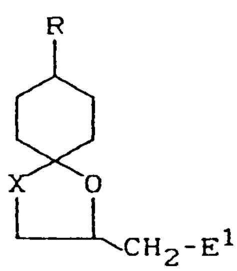

(II)

in welcher

X für Sauerstoff oder Schwefel steht,

R für Cycloalkyl mit 3 bis 7 Kohlenstoffatomen, für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht oder für einen Rest

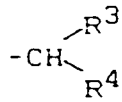

steht.

- $E^1$ für Halogen, für gegebenenfalls durch Halogen substituiertes Alkylsulfonyloxy oder für gegebenenfalls durch Alkyl mit 1 bis 4 Kohlenstoffatomen substituiertes Arylsulfonyloxy steht und
- $R^3$ für Wasserstoff, für geradkettiges oder verzweigtes Alkyl mit 1 Bis 12 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht und
- $R^4$ für geradkettiges oder verzweigtes Alkyl mit 1 bis 12 Kohlenstoffatomen, für Cycloalkyl mit 3 bis 7 Kohlenstoffatomen oder für gegebenenfalls einfach bis mehrfach, gleich oder verschieden durch geradkettiges oder verzweigtes Alkyl mit 1 bis 12 Kohlenstoffatomen substituiertes Aryl mit 6 bis 10 Kohlenstoffatomen steht, wobei jedoch $R^3$ und $R^4$ nicht gleichzeitig für Methyl stehen, dürfen.

**10.** Verfahren zur Herstellung von substituierten Heterocyclen der Formel (II) gemäß Anspruch 9, dadurch gekennzeichnet, daß man Cyclohexanonderivate der Formel (V),

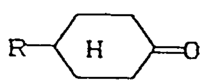

(V)

in welcher

- R die in Anspruch 9 angegebene Bedeutung hat,

mit Alkoholen der Formel (VI),

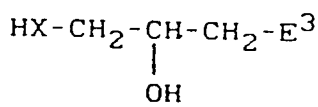

(VI)

in welcher

- X die in Anspruch 9 angegebene Bedeutung hat und
- $E^3$ für Halogen oder Hydroxy steht,

gegebenenfalls in Gegenwart eines Verdünnungsmittels und gegebenenfalls in Gegenwart eines sauren Katalysators bei Temperaturen zwischen 40 °C und 150 °C cyclisiert und in den Fällen, wo $E^3$ in Formel (VI) für eine Hydroxygruppe steht, in einer 2. Stufe die so erhältlichen Hydroxymethylheterocyclen der Formel (VII),

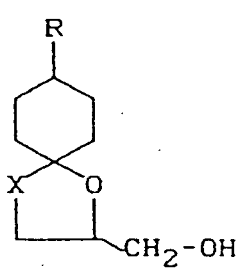

(VII)

in welcher

- X und R die in Anspruch 9 angegebene Bedeutung haben,

mit gegebenenfalls substituierten Alkyl- oder Arylsulfonylhalogeniden der Formel (VIII),

$Z\text{-}SO_2\text{-}Hal$ (VIII)

in welcher

Hal für Halogen steht und

Z für jeweils gegebenenfalls durch Halogen substituiertes Alkyl oder für gegebenenfalls durch Alkyl mit 1 bis 4 Kohlenstoffatomen substituiertes Aryl steht,

gegebenenfalls in Gegenwart eines Verdünnungsmittels und gegebenenfalls in Gegenwart eines Säurebindemittels bei Temperaturen zwischen -20 °C und +100 °C umsetzt.

11. Verwendung von substituierten Heterocyclen nach den Ansprüchen 9 und 10 als Zwischenprodukte.

12. Verwendung von Aminomethylheterocyclen der Formel (I) nach Anspruch 6 zur Bekämpfung von Schädlingen im Pflanzenschutzbereich und im Materialschutzbereich.

## Claims

1. Aminoethylheterocyclic compounds of the general formula (I)

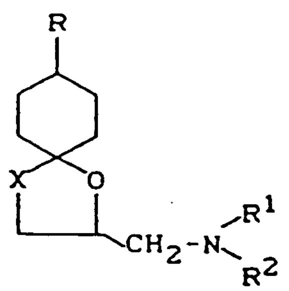

(I)

in which

X represents oxygen or sulphur,

R represents cycloalkyl having 3 to 7 carbon atoms, or represents aryl which has 6 to 10 carbon atoms and is optionally substituted by one or more identical or different straight-chain or branched alkyl radicals having 1 to 12 carbon atoms, or represents a radical

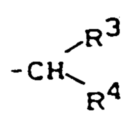

$R^1$ and $R^2$ independently of one another each represent hydrogen, or represent in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl having in each case 1 to 6 carbon atoms or hydroxyalkoxyalkyl having 2 to 6 carbon atoms in the individual alkyl parts, or represent alkoxycarbonylalkyl having 1 to 6 carbon atoms per alkoxy and alkyl part, or represent in each case straight-chain or branched dioxolanylalkyl, dioxanylalkyl or oxolanylalkyl having in each case 1 to 4 carbon atoms in the alkyl part, or represent cycloalkyl or cycloalkylalkyl having in each case 3 to 7 carbon atoms in the cycloalkyl part and if appropriate 1 to 4 carbon atoms in the straight-chain or branched alkyl part and in each case optionally substituted in the cycloalkyl part by one or more identical or different substituents, possible substituents in each case being: halogen and in each case straight-chain or branched alkyl, alkoxy, halogenoalkyl and halogenoalkoxy

having in each case 1 to 4 carbon atoms and if appropriate 1 to 9 identical or different halogen atoms;
or in addition represent arylalkyl, arylalkenyl or aryl having in each case 6 to 10 carbon atoms in the aryl part and if appropriate up to 6 carbon atoms in the straight-chain or branched alkyl or alkenyl part and in each case optionally substituted in the aryl part by one or more identical or different substituents, possible substituents on the aryl in each case being:
halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl having in each case 1 to 4 carbon atoms in the individual alkyl parts and if appropriate 1 to 9 identical or different halogen atoms, or

$R^1$ and $R^2$, together with the nitrogen atom to which they are bonded, represent a saturated 5- to 7-membered heterocyclic radical which is optionally substituted by one or more identical or different substituents and can optionally contain a further hetero atom, possible substituents being: in each case straight-chain or branched alkyl or hydroxyalkyl having in each case 1 to 4 carbon atoms,

$R^3$ represents hydrogen, or represents straight-chain or branched alkyl having 1 to 12 carbon atoms, or represents aryl which has 6 to 10 carbon atoms and is optionally substituted by one or more identical or different straight-chain or branched alkyl radicals having 1 to 12 carbon atoms and

$R^4$ represents straight-chain or branched alkyl having 1 to 12 carbon atoms, or represents cycloalkyl having 3 to 7 carbon atoms, or represents aryl which has 6 to 10 carbon atoms and is optionally substituted by one or more identical or different straight-chain or branched alkyl radicals having 1 to 12 carbon atoms,

but where $R^3$ and $R^4$ may not simultaneously represent methyl and acid addition salts thereof.

**2.** Aminomethylheterocyclic compounds according to Claim 1, wherein, in formula (I),

X represents oxygen or sulphur.

R represents cyclopentyl or cyclohexyl, or represents phenyl which is optionally substituted by one to three identical or different substituents from the group comprising methyl, ethyl, n- or i-propyl and n-, i-, s- or t-butyl, or represents a radical

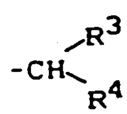

$R^1$ and $R^2$ independently of one another each represent hydrogen methyl, ethyl, n- or i-propyl, n-, i-, s- or t-butyl, n- or i-pentyl, n- or i-hexyl, n- or i-heptyl, n- or i-octyl, allyl, n- or i-butenyl, n- or i-pentenyl, propargyl, n- or i-butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl, dioxolanylethyl, dioxanylmethyl, dioxanylethyl, oxolanylmethyl or oxolanylethyl, or represent cyclopropyl, cyclopropylmethyl, cyclopropylethyl, cyclopropylpropyl, cyclopentyl, cyclopentylmethyl, cyclohexyl or cyclohexylmethyl, in each case optionally substituted by one to five identical or different substituents from the group comprising fluorine, chlorine, bromine, methyl, ethyl, n- or i-propyl, n-, i-, s- or t-butyl, trifluoromethyl and trifluoromethoxy, or represent phenyl, benzyl or phenethyl, in each case optionally substituted by one to three identical or different substituents, possible substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl or methoximinomethyl, or

$R^1$ and $R^2$ together with the nitrogen atom to which they are bonded, represent a heterocyclic radical of the formula

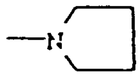 ; 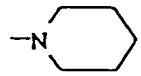 ; 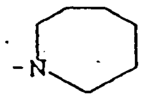 ; 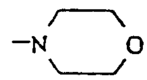

or 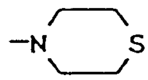

which is optionally substituted by one to three identical or different substituents, possible substituents in each case being: methyl, ethyl and hydroxymethyl,

and

$R^3$ represents hydrogen, methyl, ethyl or n- or i-propyl, or represents in each case straight-chain or branched butyl, pentyl or hexyl, or represents phenyl which is optionally substituted by one to three identical or different substituents from the group comprising methyl, ethyl, n- or i-propyl and n-, i-, s- or t- butyl and

$R^4$ represents methyl, ethyl or n- or i-propyl, or represents in each case straight-chain or branched butyl, pentyl or hexyl, or represents cyclopentyl or cyclohexyl, or represents phenyl which is optionally substituted by one to three identical or different substituents from the group comprising methyl, ethyl, n- or i-propyl and n-, i-, s- or t-butyl,

but wherein $R^3$ and $R^4$ may not simultaneously represent methyl.

**3.** Aminomethylheterocyclic compounds according to Claim 1, wherein, in the formula (I),

X represents oxygen or sulphur,

R represents cyclohexyl, or represents phenyl, or represents a radical

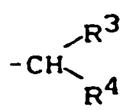

$R^1$ and $R^2$ independently of one another each represent hydrogen, methyl, ethyl, n- or i-propyl, n-, i-, s-or t-butyl, n- or i-pentyl, n- or i-hexyl, allyl, n-or i-butenyl, n- or i-pentenyl, propargyl, n- or i-butinyl, hydroxyethyl, hydroxypropyl, methoxyethyl, methoxypropyl, ethoxyethyl, ethoxypropyl, hydroxyethoxyethyl, dimethoxyethyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl, dioxolanylethyl, dioxanylmethyl, oxolanylmethyl, oxolanylethyl, cyclopropylmethyl, dichlorocyclopropylmethyl, dimethylcyclopropylmethyl, dichlorodimethylcyclopropylmethyl, cyclopentyl, cyclohexyl or cyclohexylmethyl or

$R^1$ and $R^2$, together with the nitrogen atom to which they are bonded, represent a heterocyclic radical of the formula

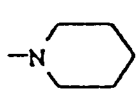 ; 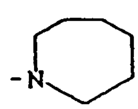 ; 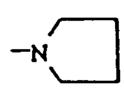 or 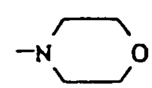

which is optionally substituted by one to three identical or different substituents, possible substituents in each case being: methyl, ethyl and hydroxymethyl, and

$R^3$ represents hydrogen, methyl, ethyl, n- or i-propyl, n-, i-, s- or t-butyl or phenyl and

$R^4$ represents methyl, ethyl, n- or i-propyl, n-, i-, s-or t-butyl, or represents cyclohexyl, or represents phenyl,

but wherein $R^3$ and $R^4$ may not simultaneously represent methyl.

**4.** Process for the preparation of aminomethylheterocyclic compounds of the general formula (I)

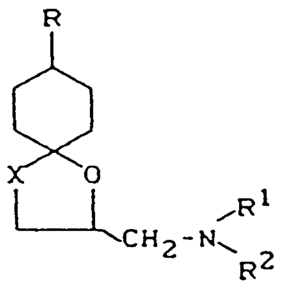

(I)

in which

X represents oxygen or suphur,

R represents cycloalkyl having 3 to 7 carbon atoms, or represents aryl which has 6 to 10 carbon atoms and is optionally substituted by one or more identical or different straight-chain or branched alkyl radicals having 1 to 12 carbon atoms, or represents a radical

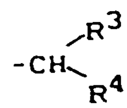

$R^1$ and $R^2$ independently of one another each represent hydrogen, or represent in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl having in each case 1 to 6 carbon atoms or hydroxyalkoxyalkyl having 2 to 6 carbon atoms in the individual alkyl parts, or represent alkoxycarbonyalkyl having 1 to 6 carbon atoms per alkoxy and alkyl part, or represent in each case straight-chain or branched dioxolanylalkyl, dioxanylalkyl or oxolanylalkyl having in each case 1 to 4 carbon atoms in the alkyl part, or represent cycloalkyl or cycloalkylalkyl having in each case 3 to 7 carbon atoms in the cycloalkyl part and if appropriate 1 to 4 carbon atoms in the straight-chain or branched alkyl part and in each case optionally substituted in the cycloalkyl part by one or more identical or different substituents, possible substituents in each case being: halogen and in each case straight-chain or branched alkyl, alkoxy, halogenoalkyl and halogenoalkoxy having in each case 1 to 4 carbon atoms and if appropriate 1 to 9 identical or different halogen atoms;

or in addition represent arylalkyl, arylalkenyl or aryl having in each case 6 to 10 carbon atoms in the aryl part and if appropriate up to 6 carbon atoms in the straight-chain or branched alkyl or alkenyl part and in each case optionally substituted in the aryl part by one or more identical or different substituents, possible substituents on the aryl in each case being:

halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl having in each case 1 to 4 carbon atoms in the individual alkyl parts and if appropriate 1 to 9 identical or different halogen atoms, or

$R^1$ and $R^2$ together with the nitrogen atom to which they are bonded, represent a saturated 5- to

7-membered heterocyclic radical which is optionally substituted by one or more identical or different substituents and can optionally contain a further hetero atom, possible substituents being: in each case straight-chain or branched alkyl or hydroxyalkyl having in each case 1 to 4 carbon atoms,

$R^3$ represents hydrogen, or represents straight-chain or branched alkyl having 1 to 12 carbon atoms, or represents aryl which has 6 to 10 carbon atoms and is optionally substituted by one or more identical or different straight-chain or branched alkyl radicals having 1 to 12 carbon atoms and

$R^4$ represents straight-chain or branched alkyl having 1 to 12 carbon atoms, or represents cycloalkyl having 3 to 7 carbon atoms, or represents aryl which has 6 to 10 carbon atoms and is optionally substituted by one or more identical or different straight-chain or branched alkyl radicals having 1 to 12 carbon atoms,

but where $R^3$ and $R^4$ may not simultaneously represent methyl and acid addition salts thereof, characterised in that

(a) substituted heterocyclic compounds of the formula (II)

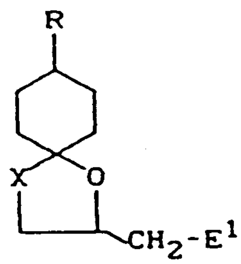

(II)

in which

R and X have the abovementioned meaning and

$E^1$ represents an electron-withdrawing leaving group, are reacted with amines of the formula (III)

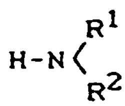

(III)

in which

$R^1$ and $R^2$ have the abovementioned meaning,

if appropriate in the presence of a diluent and if appropriate in the presence of an acid-binding agent, or by a process in which

(b) the aminomethylheterocyclic compounds obtainable by process (a), of the formula (1a)

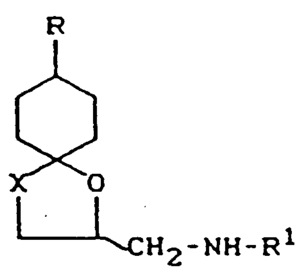

(1a)

in which

R, $R^1$ and X have the abovementioned meaning,

are reacted with alkylating agents of the formula (IV)

$R^{2-1}$-$E^2$ (IV)

in which

$R^{2-1}$ has the meaning given above for $R^2$ with the exception of aryl and hydrogen and

$E^2$ represents an electron-withdrawing leaving group,

if appropriate in the presence of a diluent and if appropriate in the presence of an acid-binding agent, and, if appropriate, an acid is then added on or the reaction is followed by a physical resolution method.

**5.** Agents for combating pests, characterised in that they contain at least one aminomethylheterocyclic compound of the formula (I) according to Claims 1 to 4.

**6.** Use of aminomethylheterocyclic compounds of the formula (I) according to Claims 1 to 4, for combating pests.

**7.** Method of combating pests, characterised in that aminomethylheterocyclic compounds of the formula (I) according to Claims 1 to 4 are allowed to act on pests and/or their environment.

**8.** Process for the preparation of agents for combating pests, characterised in that aminomethylheterocyclic compounds of the formula (I) according to Claims 1 to 4 are mixed with extenders and/or surface-active agents.

**9.** Substituted heterocyclic compounds of the formula (II)

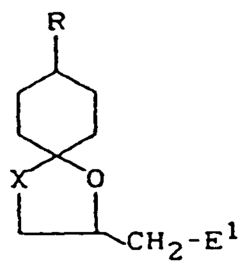

(II)

in which

X represents oxygen or sulphur,

R represents cycloalkyl with 3 to 7 carbon atoms, or represents aryl which has 6 to 10 carbon atoms and is optionally substituted by one or more identical or different straight-chain or branched alkyl radicals having 1 to 12 carbon atoms, or represents a radical

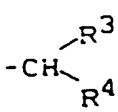

$E^1$ represents halogen, or represents alkylsulphonyloxy which is optionally substituted by halogen, or represents arylsulphonyloxy which is optionally substituted by alkyl having 1 to 4 carbon atoms and

$R^3$ represents hydrogen, or represents straight-chain or branched alkyl having 1 to 12 carbon atoms, or represents aryl which has 6 to 10 carbon atoms and is optionally substituted by one or more identical or different straight-chain or branched alkyl radicals having 1 to 12 carbon atoms and

$R^4$ represents straight-chain or branched alkyl having 1 to 12 carbon atoms, or represents cycloalkyl having 3 to 7 carbon atoms, or represents aryl which has 6 to 10 carbon atoms

and is optionally substituted by one or more identical or different straight-chain or branched alkyl radicals having 1 to 12 carbon atoms, but wherein $R^3$ and $R^4$ may not simultaneously represent methyl.

**10.** Process for the preparation of substituted heterocyclic compounds of the formula (II) according to Claim 9, characterised in that cyclohexanone derivatives of the formula (V)

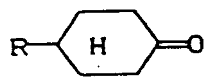

(V)

in which

R has the meaning given in Claim 9,

are cyclized with alcohols of the formula (VI)

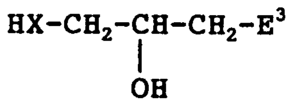

(VI)

in which

X has the meaning given in Claim 9 and

$E^3$ represents halogen or hydroxyl,

if appropriate in the presence of a diluent and if appropriate in the presence of an acid catalyst, at temperatures between 40 °C and 150 °C, and in the cases where $E^3$ in formula (VI) represents a hydroxyl group, the hydroxymethylheterocyclic compounds thus obtainable, of the formula (VII)

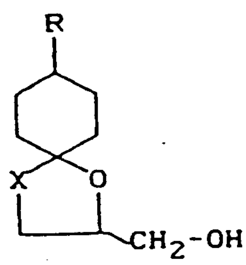

(VII)

in which

X and R have the meaning given in Claim 9,

are reacted in a 2nd stage with optionally substituted alkyl- or arylsulphonyl halides of the formula (VIII)

$Z-SO_2-Hal$ (VIII)

in which

Hal represents halogen and

Z represents alkyl which is in each case optionally substituted by halogen, or represents aryl which is optionally substituted by alkyl having 1 to 4 carbon atoms,

if appropriate in the presence of a diluent and if appropriate in the presence of an acid-binding agent at temperatures between -20 °C and +100 °C.

**11.** Use of substituted heterocyclic compounds according to Claims 9 and 10 as intermediate products.

**12.** Use of aminomethylheterocyclic compounds of the formula (I) according to Claim 6 for combating pests in the plant protection field and in the field of preservation of materials.

**Revendications**

**1.** Composés hétérocycliques aminométhylés répondant à la formule générale (I)

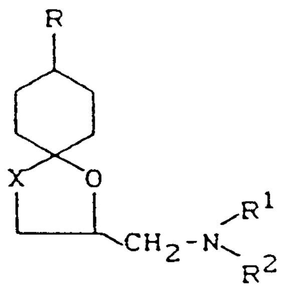

(I)

dans laquelle

X représente un atome d'oxygène ou un atome de soufre,

R représente un groupe cycloalkyle contenant de 3 à 7 atomes de carbone, un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, ou représente un radical

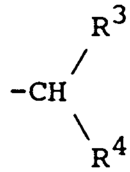

$R^1$ et $R^2$ représentent chacun, indépendamment l'un de l'autre, un atome d'hydrogène; un groupe alkyle contenant de 1 à 12 atomes de carbone, un groupe alcényle contenant de 3 à 8 atomes de carbone, un groupe alcynyle contenant de 3 à 8 atomes de carbone, un groupe hydroxyalkyle contenant de 2 à 6 atomes de carbone, un groupe alcoxyalkyle ou dialcoxyalkyle contenant chacun de 1 à 6 atomes de carbone ou un groupe hydroxyalcoxyalkyle contenant de 2 à 6 atomes de carbone dans les fractions alkyle individuelles, ces groupes étant chacun à chaîne droite ou ramifiée; un groupe alcoxycarbonylalkyle contenant de 1 à 6 atomes de carbone, chaque fois, dans la fraction alcoxy ou alkyle; un groupe dioxolanylalkyle, un groupe dioxanylalkyle ou un groupe oxolanylalkyle, chaque fois à chaîne droite ou ramifiée, contenant chacun de 1 à 4 atomes de carbone dans la fraction alkyle; ou encore un groupe cycloalkyle ou cycloalkylalkyle, chaque fois éventuellement substitué de manière identique ou différente, de une à plusieurs fois, dans la fraction cycloalkyle, contenant chacun de 3 à 7 atomes de carbone dans la fraction cycloalkyle et éventuellement de 1 à 4 atomes de carbone dans la fraction alkyle à chaîne droite ou ramifiée, groupes dans lesquels, comme substituants, entrent chaque fois en ligne de compte :

un atome d'halogène, un groupe alkyle, un groupe alcoxy, un groupe halogénalkyle ou un groupe halogénalcoxy, chaque fois à chaîne droite ou ramifiée, contenant chacun de 1 à 4 atomes de carbone et éventuellement de 1 à 9 atomes d'halogène identiques ou différents;

en outre, un groupe arylalkyle, un groupe arylalcényle ou un groupe aryle, chaque fois éventuellement substitué une à plusieurs-fois, de manière identique ou différente dans la fraction aryle, contenant chacun de 6 à 10 atomes de carbone dans la fraction aryle et éventuellement jusqu'à 6 atomes de carbone dans la fraction alkyle ou alcényle à

chaîne droite ou ramifiée, dans lesquels, comme substituants du radical aryle, on peut chaque fois envisager :
un atome d'halogène, un groupe cyano, un groupe nitro, un groupe alkyle, un groupe alcoxy, un groupe alkylthio, un groupe halogénalkyle, un groupe halogénalcoxy, un groupe halogénalkylthio, un groupe alcoxycarbonyle ou un groupe alcoximinoalkyle chaque fois à chaîne droite ou ramifiée, contenant chacun de 1 à 4 atomes de carbone dans les fractions alkyle individuelles et éventuellement de 1 à 9 atomes d'halogène identiques ou différents, ou bien

$R^1$ et $R^2$ représentent, conjointement avec l'atome d'azote auquel ils sont liés, un hétérocycle penta- à heptagonal saturé, éventuellement substitué de une à plusieurs fois de manière identique ou différente, qui peut contenir éventuellement un autre hétéroatome, dans lequel, comme substituants, on envisage : un groupe alkyle ou un groupe hydroxyalkyle chacun à chaîne droite ou ramifiée, contenant chacun de 1 à 4 atomes de carbone,

$R^3$ représente un atome d'hydrogène, un groupe alkyle à chaîne droite ou ramifiée contenant de 1 à 12 atomes de carbone, ou un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, et

$R^4$ représente un groupe alkyle à chaîne droite ou ramifiée contenant de 1 à 12 atomes de carbone, un groupe cycloalkyle contenant de 3 à 7 atomes de carbone, ou un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone,

$R^3$ et $R^4$ ne pouvant pas représenter en même temps un groupe méthyle, ainsi que leurs sels d'addition d'acides.

**2.** Composés hétérocycliques aminométhylés selon la revendication 1, dans lesquels, dans la formule (I),

X représente un atome d'oxygène ou un atome de soufre

R représente un groupe cyclopentyle, un groupe cyclohexyle ou un groupe phényle éventuellement substitué de 1 à 3 fois de manière identique ou différente par un groupe méthyle, un groupe éthyle, un groupe n- ou i- propyle, un groupe n-, i-, s- ou t-butyle, ou représente le radical

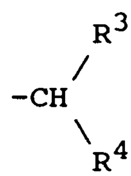

$R^1$ et $R^2$ représentent chacun, indépendamment l'un de l'autre, un atome d'hydrogène, un groupe méthyle, un groupe éthyle, un groupe n- ou i-propyle, un groupe n-, i-, s- ou t-butyle, un groupe n- ou i-pentyle, un groupe n- ou i-hexyle, un groupe n- ou i-heptyle, un groupe n- ou i-octyle, un groupe allyle, un groupe n- ou i-buténыle, un groupe n- ou i-penténylе, un groupe propargyle, un groupe n- ou i-butynyle, un groupe hydroxyéthyle, un groupe hydroxypropyle, un groupe méthoxyéthyle, un groupe éthoxyéthyle, un groupe propoxyéthyle, un groupe butoxyéthyle, un groupe méthoxypropyle, un groupe éthoxypropyle, un groupe propoxypropyle, un groupe butoxypropyle, un groupe hydroxyéthoxyéthyle, un groupe diméthoxyéthyle, un groupe diméthoxypropyle, un groupe diéthoxyéthyle, un groupe méthoxycarbonylméthyle, un groupe méthoxycarbonyléthyle, un groupe méthoxycarbonylpropyle, un groupe éthoxycarbonylméthyle, un groupe éthoxycarbonyléthyle, un groupe éthoxycarbonylpropyle, un groupe propoxycarbonylméthyle, un groupe propoxycarbonyléthyle, un groupe propoxycarbonylpropyle, un groupe dioxolanylméthyle, un groupe dioxolanyléthyle, un groupe dioxanylméthyle, un groupe dioxanyléthyle, un groupe oxolanylméthyle, un groupe oxolanyléthyle; un groupe cyclopropyle, un groupe cyclopropylméthyle, un groupe cyclopropyléthyle, un groupe

cyclopropylpropyle, un groupe cyclopentyle, un groupe cyclopentylméthyle, un groupe cyclohexyle ou un groupe cyclohexylméthyle, chacun éventuellement substitué de 1 à 5 fois, de manière identique ou différente, par un atome de fluor, un atome de chlore, un atome de brome, un groupe méthyle, un groupe éthyle, un groupe n- ou i-propyle, un groupe n-, i-, s- ou t-butyle, un groupe trifluorométhyle ou un groupe trifluorométhoxy; ou encore un groupe phényle, un groupe benzyle ou un groupe phényléthyle, chacun éventuellement substitué de 1 à 3 fois, de manière identique ou différente, dans lesquels, comme substituants, entrent chaque fois en ligne de compte :
un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe méthoxycarbonyle, un groupe éthoxycarbonyle ou un groupe méthoximinométhyle, ou bien

$R^1$ et $R^2$ représentent, conjointement avec l'atome d'azote auquel ils sont liés, un hétérocycle chaque fois éventuellement substitué de 1 à 3 fois de manière identique ou différente, répondant à la formule

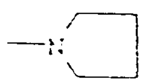 ; 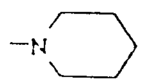 ; 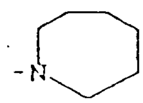 ; 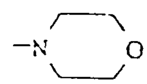

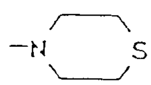

ou

dans lesquelles, comme substituants, on envisage : un groupe méthyle, un groupe éthyle ou un groupe hydroxyméthyle,

et

$R^3$ représente un atome d'hydrogène, un groupe méthyle, un groupe éthyle, un groupe n- ou i-propyle; un groupe butyle, un groupe pentyle ou un groupe hexyle, chacun de ces groupes étant à chaîne droite ou ramifiée, ou représente un groupe phényle éventuellement substitué de 1 à 3 fois, de manière identique ou différente par un groupe méthyle, un groupe éthyle, un groupe n- ou i-propyle ou par un groupe n-, i-, s- ou t-butyle, et

$R^4$ représente un groupe méthyle, un groupe éthyle, un groupe n- ou i-propyle; un groupe butyle, un groupe pentyle ou un groupe hexyle, chacun de ces groupes étant à chaîne droite ou ramifiée; un groupe cyclopentyle, un groupe cyclohexyle, ou représente un groupe phényle éventuellement substitué de 1 à 3 fois, de manière identique ou différente par un groupe méthyle, un groupe éthyle, un groupe n- ou i-propyle ou par un groupe n-, i-, s- ou t-butyle,

$R^3$ et $R^4$ ne pouvant toutefois représenter en même temps un groupe méthyle.

**3.** Composés hétérocycliques aminométhylés selon la revendication 1, dans lesquels, dans la formule (I),

X représente un atome d'oxygène ou un atome de soufre

R représente un groupe cyclohexyle, un groupe phényle ou le radical

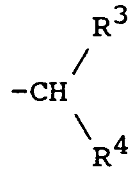

$R^1$ et $R^2$ représentent chacun, indépendamment l'un de l'autre, un atome d'hydrogène, un groupe méthyle, un groupe éthyle, un groupe n- ou i-propyle, un groupe n-, i-, s- ou t-butyle, un groupe n- ou i-pentyle, un groupe n- ou i-hexyle, un groupe allyle, un groupe n- ou i-butényle, un groupe n- ou i-pentényle, un groupe propargyle, un groupe n- ou i-butynyle, un groupe hydroxyéthyle, un groupe hydroxypropyle, un groupe méthoxyéthyle, un groupe méthoxypropyle, un groupe éthoxyéthyle, un groupe éthoxypropyle, un groupe hydroxyéthoxyéthyle, un groupe diméthoxyéthyle, un groupe diéthoxyéthyle, un groupe méthoxycarbonylméthyle, un groupe méthoxycarbonyléthyle, un groupe méthoxycarbonylpropyle, un groupe éthoxycarbonylméthyle, un groupe éthoxycarbonyléthyle, un groupe éthoxycarbonylpropyle, un groupe propoxycarbonylméthyle, un groupe propoxycarbonyléthyle, un groupe propoxycarbonylpropyle, un groupe dioxolanylméthyle, un groupe dioxolanyléthyle, un groupe dioxanylméthyle, un groupe oxolanylméthyle, un groupe oxolanyléthyle, un groupe cyclopropylméthyle, un groupe dichlorocyclopropylméthyle, un groupe diméthylcyclopropylméthyle, un groupe dichlorodiméthylcyclopropylméthyle, un groupe cyclopentyle, un groupe cyclohexyle ou un groupe cyclohexylméthyle, ou bien

$R^1$ et $R^2$ représentent, conjointement avec l'atome d'azote auquel ils sont liés, un hétérocycle éventuellement substitué de 1 à 3 fois, de manière identique ou différente, répondant à la formule

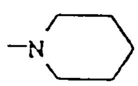 ; 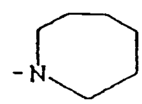 ; 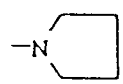 ou 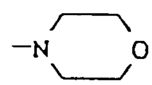

dans lesquelles, comme substituants, on envisage chaque fois un groupe méthyle, un groupe éthyle ou un groupe hydroxyméthyle, et

$R^3$ représente un atome d'hydrogène, un groupe méthyle, un groupe éthyle, un groupe n- ou i-propyle, un groupe n-, i-, s- ou t-butyle ou encore un groupe phényle, et

$R^4$ représente un groupe méthyle, un groupe éthyle, un groupe n- ou i-propyle, un groupe n-, i-, s- ou t-butyle; un groupe cyclohexyle ou un groupe phényle,

dans lesquels, toutefois, $R^3$ et $R^4$ ne peuvent représenter en même temps un groupe méthyle.

4. Procédé pour la préparation de composés hétérocycliques aminométhylés répondant à la formule générale (I)

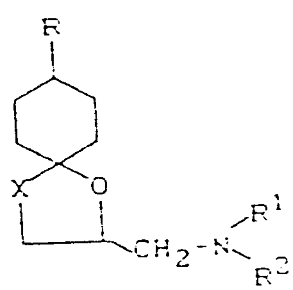

(I)

dans laquelle

X représente un atome d'oxygène ou un atome de soufre,

R représente un groupe cycloalkyle contenant de 3 à 7 atomes de carbone, un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, ou représente un radical

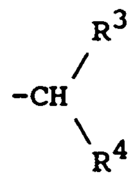

$R^1$ et $R^2$ représentent chacun, indépendamment l'un de l'autre, un atome d'hydrogène; un groupe alkyle contenant de 1 à 12 atomes de carbone, un groupe alcényle contenant de 3 à 8 atomes de carbone, un groupe alcynyle contenant de 3 à 8 atomes de carbone, un groupe hydroxyalkyle contenant de 2 à 6 atomes de carbone, un groupe alcoxyalkyle ou dialcoxyalkyle contenant chacun de 1 à 6 atomes de carbone ou un groupe hydroxyalcoxyalkyle contenant de 2 à 6 atomes de carbone dans les fractions alkyle individuelles, ces groupes étant chacun à chaîne droite ou ramifiée; un groupe alcoxycarbonylalkyle contenant de 1 à 6 atomes de carbone, chaque fois, dans la fraction alcoxy ou alkyle; un groupe dioxolanylalkyle, un groupe dioxanylalkyle ou un groupe oxolanylalkyle, chaque fois à chaîne droite ou ramifiée, contenant chacun de 1 à 4 atomes de carbone dans la fraction alkyle; ou encore un groupe cycloalkyle ou cycloalkylalkyle, chaque fois éventuellement substitué de manière identique ou différente, de une à plusieurs fois, dans la fraction cycloalkyle, contenant chacun de 3 à 7 atomes de carbone dans la fraction cycloalkyle et éventuellement de 1 à 4 atomes de carbone dans la fraction alkyle à chaîne droite ou ramifiée, groupes dans lesquels, comme substituants, entrent chaque fois en ligne de compte :

un atome d'halogène, un groupe alkyle, un groupe alcoxy, un groupe halogénalkyle ou un groupe halogénalcoxy, chaque fois à chaîne droite ou ramifiée, contenant chacun de 1 à 4 atomes de carbone et éventuellement 1 à 9 atomes d'halogène identiques ou différents; en outre, un groupe arylalkyle, un groupe arylalcényle ou un groupe aryle, chaque fois éventuellement substitué de une à plusieurs fois, de manière identique ou différente, dans la fraction aryle, contenant chacun de 6 à 10 atomes de carbone dans la fraction aryle et éventuellement jusqu'à 6 atomes de carbone dans la fraction alkyle ou alcényle à chaîne droite ou ramifiée; groupes dans lesquels, comme substituants du groupe aryle, on peut chaque fois envisager :

un atome d'halogène, un groupe cyano, un groupe nitro, un groupe alkyle, un groupe alcoxy, un groupe alkylthio, un groupe halogénalkyle, un groupe halogénalcoxy, un groupe halogénalkylthio, un groupe alcoxycarbonyle ou un groupe alcoximinoalkyle chaque fois à chaîne droite ou ramifiée, contenant chacun de 1 à 4 atomes de carbone dans les fractions alkyle individuelles et éventuellement de 1 à 9 atomes d'halogène identiques ou différents, ou bien

$R^1$ et $R^2$ représentent, conjointement avec l'atome d'azote auquel ils sont liés, un hétérocycle penta- à heptagonal saturé, éventuellement substitué de une à plusieurs fois de manière identique ou différente, qui peut contenir éventuellement un autre hétéroatome, dans lequel, comme substituants, on envisage :

un groupe alkyle ou hydroxyalkyle chaque fois à chaîne droite ou ramifiée, contenant chacun de 1 à 4 atomes de carbone,

$R^3$ représente un atome d'hydrogène, un groupe alkyle à chaîne droite ou ramifiée contenant de 1 à 12 atomes de carbone, ou un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, et

$R^4$ représente un groupe alkyle à chaîne droite ou ramifiée contenant de 1 à 12 atomes de carbone, un groupe cycloalkyle contenant de 3 à 7 atomes de carbone, ou un groupe

aryle contenant de 6 à 10 atomes de carbone éventuellement substitué de une à plusieurs fois de manière identique ou différente par un groupe alkyle à chaîne droite ou ramifiée contenant de 1 à 12 atomes de carbone

$R^3$ et $R^4$ ne pouvant toutefois pas représenter en même temps un groupe méthyle,

ainsi que leurs sels d'addition d'acides, caractérisé en ce qu'on fait réagir

(a) des composés hétérocycliques substitués de formule (II)

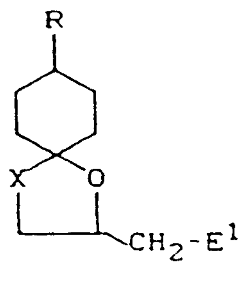

(II)

dans laquelle

R et X ont la signification mentionnée ci-dessus et

$E^1$ représente un groupe qui se sépare attirant les électrons,

avec des amines de formule (III)

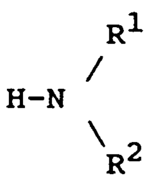

dans laquelle

$R^1$ et $R^2$ ont la signification mentionnée ci-dessus,

éventuellement en présence d'un diluant et éventuellement en présence d'un agent neutralisateur d'acides, ou

(b) en ce qu'on fait réagir les composés hétérocycliques aminométhylés que l'on peut obtenir par le procédé (a) et qui répondent à la formule (Ia)

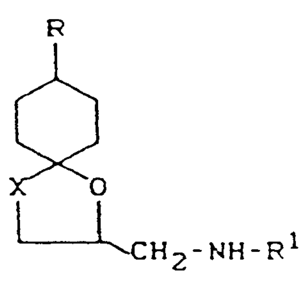

(Ia)

dans laquelle R, $R^1$ et X ont la signification mentionnée ci-dessus,

avec des agents d'alkylation de formule (IV)

$R^{2-1}$-$E^2$ (IV)

dans laquelle

$R^{2-1}$ a la signification indiquée ci-dessus pour $R^2$, à l'exception du groupe aryle et un atome d'hydrogène

et

$E^2$ représente un groupe qui se sépare attirant les électrons,

éventuellement en présence d'un diluant et éventuellement en présence d'un agent neutralisateur d'acides et ensuite, on ajoute éventuellement un acide ou bien on met en oeuvre un procédé physique de séparation.

**5.** Agent pour la lutte contre les parasites, caractérisé en ce qu'il contient au moins un composé hétérocyclique aminométhylé de formule (I) selon les revendications 1 à 4.

**6.** Utilisation de composés hétérocycliques aminométhylés de formule (I) selon les revendications 1 à 4, pour lutter contre les parasites.

**7.** Procédé pour la lutte contre les parasites, caractérisé en ce qu'on laisse agir des composés hétérocycliques aminométhylés de formule (I) selon les revendications 1 à 4, sur des parasites et/ou dans leur biotope.

**8.** Procédé pour la préparation d'agents pour la lutte contre les parasites, caractérisé en ce qu'on mélange des composés hétérocycliques aminométhylés de formule (I) selon les revendications 1 à 4, avec des diluants et/ou des agents tensio-actifs.

**9.** Composés hétérocycliques substitués de formule (II)

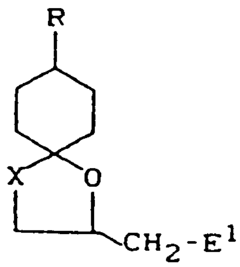

(II)

dans laquelle

X représente un atome d'oxygène ou un atome de soufre

R représente un groupe cycloalkyle contenant de 3 à 7 atomes de carbone, un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, ou représente un radical

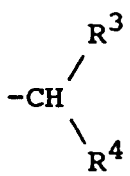

$E^1$ représente un atome d'halogène, un groupe alkylsulfonyloxy éventuellement substitué par un atome d'halogène, ou un groupe arylsulfonyloxy éventuellement substitué par un groupe alkyle contenant de 1 à 4 atomes de carbone, et

$R^3$ représente un atome d'hydrogène, un groupe alkyle à chaîne droite ou ramifiée contenant de 1 à 12 atomes de carbone, ou un groupe aryle contenant de 6 à 10 atomes de carbone éventuellement substitué de une à plusieurs fois de manière identique ou différente par un groupe alkyle à chaîne droite ou ramifiée contenant de 1 à 12 atomes de carbone, et

$R^4$ représente un groupe alkyle à chaîne droite ou ramifiée contenant de 1 à 12 atomes de carbone, un groupe cycloalkyle contenant de 3 à 7 atomes de carbone, ou un groupe aryle

contenant de 6 à 10 atomes de carbone éventuellement substitué de une à plusieurs fois de manière identique ou différente par un groupe alkyle à chaîne droite ou ramifiée contenant de 1 à 12 atomes de carbone, $R^3$ et $R^4$ ne pouvant toutefois pas représenter en même temps un groupe méthyle.

**10.** Procédé pour la préparation de composés hétérocycliques substitués de formule (II) selon la revendication 9, caractérisé en ce qu'on fait réagir des dérivés de cyclohexanone de formule (V)

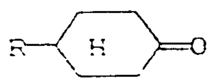 (V)

dans laquelle

R a la signification indiquée à la revendication 9,

avec des alcools de formule (VI)

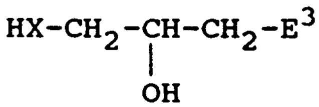 (VI)

dans laquelle

X a la signification indiquée à la revendication 9, et

$E^3$ représente un atome d'halogène ou un groupe hydroxyle

éventuellement en présence d'un diluant et on cyclise éventuellement en présence d'un catalyseur acide à des températures entre 40 ° C et 150 ° C et, dans les cas ou $E^3$ dans la formule (VI) représente un groupe hydroxyle, au cours d'une deuxième étape, on fait réagir les composés hétérocycliques hydroxyméthylés de formule (VII) que l'on peut obtenir de cette manière

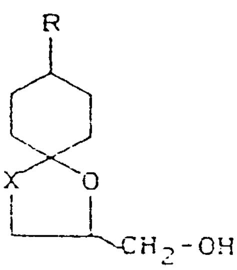 (VII)

dans laquelle

X et R ont la signification indiquée à la revendication 9

avec des halogénures d'alkyl- ou d'aryl-sulfonyle éventuellement substitués de formule (VIII)

$Z\text{-}SO_2\text{-}Hal$ (VIII)

dans laquelle

Hal représente un atome d'halogène, et

Z représente un groupe alkyle éventuellement substitué par un atome d'halogène ou bien un groupe aryle éventuellement substitué par un groupe alkyle contenant de 1 à 4 atomes de carbone;

éventuellement en présence d'un diluant et éventuellement en présence d'un agent neutralisateur d'acides à des températures entre -20 ° C et +100 ° C.

**11.** Utilisation de composés hétérocycliques substitués selon les revendications 9 et 10, comme produits intermédiaires.

**12.** Utilisation de composés hétérocycliques aminométhylés répondant à la formule (I) selon la revendication 6, pour lutter contre les parasites dans le domaine de la protection des plantes et dans le domaine de la protection des matériaux.